# EUROPEAN PATENT APPLICATION

(11) **EP 3 502 701 A2**
(43) Date of publication of application: **26.06.2019**
(21) Application number: 19154036.8
(22) Date of filing: 09.12.2016
(51) Int. Cl.: G01N 33/574

(54) **REAGENTS AND METHODS FOR MONITORING BREAST CANCER THERAPY**

(30) Priority: 11.12.2015 US 201562266189 P
(62) Divisional of application: 16822567.0
(71) Applicant: Sanford Health, Sioux Falls, SD 57105 (US)
(72) Inventor: EGLAND, Kristi A., Sioux Falls, SD 57105 (US); EVANS, Rick L., Sioux Falls, SD 57105 (US); POTTALA, James V., Sioux Falls, SD 57105 (US)
(74) Representative: Kehl, Ascherl, Liebhoff & Ettmayr Patentanwälte Partnerschaft mbB

(57) **Abstract**

Disclosed herein are compositions including reagents for detecting human autoantibodies against at least two proteins selected from the group consisting of A1AT, ANGPTL4, LRP10, GFRA1, LGALS3, CST2, DKK1, CAPC, GRP78, and GRN, wherein at least one of the antibody detection markers is selected from the group consisting of A1AT, LGALS3, and CAPC, and their use in monitoring efficacy of breast cancer therapy and breast cancer recurrence.

SEQUENCE LISTING

<110> Sanford Health

Egland, Kristi

Evans, Rick

Pottala, James V.

<120> Reagents and Methods for Monitoring Breast Cancer Therapy

<130> 15-460-PCT

<150> 62/266189

<151> 2015-12-11

<160> 12

<170> PatentIn version 3.5

<210> 1

<211> 376

<212> PRT

<213> Homo sapiens

<400> 1

Lys Ser Pro Arg Phe Ala Ser Trp Asp Glu Met Asn Val Leu Ala His

1               5                   10                  15

Gly Leu Leu Gln Leu Gly Gln Gly Leu Arg Glu His Ala Glu Arg Thr

            20                  25                  30

Arg Ser Gln Leu Ser Ala Leu Glu Arg Arg Leu Ser Ala Cys Gly Ser
        35                  40                  45
Ala Cys Gln Gly Thr Glu Gly Ser Thr Asp Leu Pro Leu Ala Pro Glu
    50                  55                  60
Ser Arg Val Asp Pro Glu Val Leu His Ser Leu Gln Thr Gln Leu Lys
65                  70                  75                  80
Ala Gln Asn Ser Arg Ile Gln Gln Leu Phe His Lys Val Ala Gln Gln
                85                  90                  95
Gln Arg His Leu Glu Lys Gln His Leu Arg Ile Gln His Leu Gln Ser
            100                 105                 110
Gln Phe Gly Leu Leu Asp His Lys His Leu Asp His Glu Val Ala Lys
        115                 120                 125
Pro Ala Arg Arg Lys Arg Leu Pro Glu Met Ala Gln Pro Val Asp Pro
    130                 135                 140

Ala His Asn Val Ser Arg Leu His Arg Leu Pro Arg Asp Cys Gln Glu
145                 150                 155                 160

Leu Phe Gln Val Gly Glu Arg Gln Ser Gly Leu Phe Glu Ile Gln Pro
                165                 170                 175

Gln Gly Ser Pro Pro Phe Leu Val Asn Cys Lys Met Thr Ser Asp Gly
            180                 185                 190

Gly Trp Thr Val Ile Gln Arg Arg His Asp Gly Ser Val Asp Phe Asn
        195                 200                 205

Arg Pro Trp Glu Ala Tyr Lys Ala Gly Phe Gly Asp Pro His Gly Glu
    210                 215                 220

Phe Trp Leu Gly Leu Glu Lys Val His Ser Ile Thr Gly Asp Arg Asn
225                 230                 235                 240

Ser Arg Leu Ala Val Gln Leu Arg Asp Trp Asp Gly Asn Ala Glu Leu
                245                 250                 255

Leu Gln Phe Ser Val His Leu Gly Gly Glu Asp Thr Ala Tyr Ser Leu
            260                 265                 270

Gln Leu Thr Ala Pro Val Ala Gly Gln Leu Gly Ala Thr Thr Val Pro
        275                 280                 285

Pro Ser Gly Leu Ser Val Pro Phe Ser Thr Trp Asp Gln Asp His Asp
    290                 295                 300

Leu Arg Arg Asp Lys Asn Cys Ala Lys Ser Leu Ser Gly Gly Trp Trp
305                 310                 315                 320

Phe Gly Thr Cys Ser His Ser Asn Leu Asn Gly Gln Tyr Phe Arg Ser
                325                 330                 335

Ile Pro Gln Gln Arg Gln Lys Leu Lys Lys Gly Ile Phe Trp Lys Thr
            340                 345                 350

Trp Arg Gly Arg Tyr Tyr Pro Leu Gln Ala Thr Thr Met Leu Ile Gln
        355                 360                 365

Pro Met Ala Ala Glu Ala Ala Ser
    370                 375

<210> 2

<211>  238

<212>  PRT

<213>  Homo sapiens

<400>  2

Val Ser Ala Thr Leu Asn Ser Val Leu Asn Ser Asn Ala Ile Lys Asn

1               5                   10                  15

Leu Pro Pro Pro Leu Gly Gly Ala Ala Gly His Pro Gly Ser Ala Val

            20                  25                  30

Ser Ala Ala Pro Gly Ile Leu Tyr Pro Gly Gly Asn Lys Tyr Gln Thr

        35                  40                  45

Ile Asp Asn Tyr Gln Pro Tyr Pro Cys Ala Glu Asp Glu Glu Cys Gly

    50                  55                  60

Thr Asp Glu Tyr Cys Ala Ser Pro Thr Arg Gly Gly Asp Ala Gly Val

65                  70                  75                  80

Gln Ile Cys Leu Ala Cys Arg Lys Arg Arg Lys Arg Cys Met Arg His

                85                  90                  95

Ala Met Cys Cys Pro Gly Asn Tyr Cys Lys Asn Gly Ile Cys Val Ser

            100                 105                 110

Ser Asp Gln Asn His Phe Arg Gly Glu Ile Glu Glu Thr Ile Thr Glu
        115                 120                 125

Ser Phe Gly Asn Asp His Ser Thr Leu Asp Gly Tyr Ser Arg Arg Thr
    130                 135                 140

Thr Leu Ser Ser Lys Met Tyr His Thr Lys Gly Gln Glu Gly Ser Val
145                 150                 155                 160

Cys Leu Arg Ser Ser Asp Cys Ala Ser Gly Leu Cys Cys Ala Arg His
                165                 170                 175

Phe Trp Ser Lys Ile Cys Lys Pro Val Leu Lys Glu Gly Gln Val Cys
            180                 185                 190

Thr Lys His Arg Arg Lys Gly Ser His Gly Leu Glu Ile Phe Gln Arg
        195                 200                 205

Cys Tyr Cys Gly Glu Gly Leu Ser Cys Arg Ile Gln Lys Asp His His
    210                 215                 220

Gln Ala Ser Asn Ser Ser Arg Leu His Thr Cys Gln Arg His
225                 230                 235

<210>  3
<211>  441
<212>  PRT
<213>  Homo sapiens

<400>  3

Asp Arg Leu Asp Cys Val Lys Ala Ser Asp Gln Cys Leu Lys Glu Gln
1               5                   10                  15

Ser Cys Ser Thr Lys Tyr Arg Thr Leu Arg Gln Cys Val Ala Gly Lys
            20                  25                  30

Glu Thr Asn Phe Ser Leu Ala Ser Gly Leu Glu Ala Lys Asp Glu Cys
        35                  40                  45

Arg Ser Ala Met Glu Ala Leu Lys Gln Lys Ser Leu Tyr Asn Cys Arg
    50                  55                  60

Cys Lys Arg Gly Met Lys Lys Glu Lys Asn Cys Leu Arg Ile Tyr Trp
65                  70                  75                  80

Ser Met Tyr Gln Ser Leu Gln Gly Asn Asp Leu Leu Glu Asp Ser Pro
                85                  90                  95
Tyr Glu Pro Val Asn Ser Arg Leu Ser Asp Ile Phe Arg Val Val Pro
            100                 105                 110
Phe Ile Ser Asp Val Phe Gln Gln Val Glu His Ile Pro Lys Gly Asn
        115                 120                 125
Asn Cys Leu Asp Ala Ala Lys Ala Cys Asn Leu Asp Asp Ile Cys Lys
    130                 135                 140
Lys Tyr Arg Ser Ala Tyr Ile Thr Pro Cys Thr Thr Ser Val Ser Asn
145                 150                 155                 160
Asp Val Cys Asn Arg Arg Lys Cys His Lys Ala Leu Arg Gln Phe Phe
                165                 170                 175
Asp Lys Val Pro Ala Lys His Ser Tyr Gly Met Leu Phe Cys Ser Cys
            180                 185                 190
Arg Asp Ile Ala Cys Thr Glu Arg Arg Arg Gln Thr Ile Val Pro Val

        195                 200                 205

Cys Ser Tyr Glu Glu Arg Glu Lys Pro Asn Cys Leu Asn Leu Gln Asp
    210                 215                 220

Ser Cys Lys Thr Asn Tyr Ile Cys Arg Ser Arg Leu Ala Asp Phe Phe
225                 230                 235                 240

Thr Asn Cys Gln Pro Glu Ser Arg Ser Val Ser Ser Cys Leu Lys Glu
                245                 250                 255

Asn Tyr Ala Asp Cys Leu Leu Ala Tyr Ser Gly Leu Ile Gly Thr Val
            260                 265                 270

Met Thr Pro Asn Tyr Ile Asp Ser Ser Ser Leu Ser Val Ala Pro Trp
        275                 280                 285

Cys Asp Cys Ser Asn Ser Gly Asn Asp Leu Glu Glu Cys Leu Lys Phe
    290                 295                 300

Leu Asn Phe Phe Lys Asp Asn Thr Cys Leu Lys Asn Ala Ile Gln Ala
305                 310                 315                 320

Phe Gly Asn Gly Ser Asp Val Thr Val Trp Gln Pro Ala Phe Pro Val
                325                 330                 335

Gln Thr Thr Thr Ala Thr Thr Thr Thr Ala Leu Arg Val Lys Asn Lys
            340                 345                 350

Pro Leu Gly Pro Ala Gly Ser Glu Asn Glu Ile Pro Thr His Val Leu
        355                 360                 365

Pro Pro Cys Ala Asn Leu Gln Ala Gln Lys Leu Lys Ser Asn Val Ser
    370                 375                 380

Gly Asn Thr His Leu Cys Ile Ser Asn Gly Asn Tyr Glu Lys Glu Gly
385                 390                 395                 400

Leu Gly Ala Ser Ser His Ile Thr Thr Lys Ser Met Ala Ala Pro Pro
                405                 410                 415

Ser Cys Gly Leu Ser Pro Leu Leu Val Leu Val Val Thr Ala Leu Ser
            420                 425                 430

Thr Leu Leu Ser Leu Thr Glu Thr Ser
        435                 440

<210>  4

<211>  576

<212>  PRT

<213>  Homo sapiens

<400>  4

Thr Arg Cys Pro Asp Gly Gln Phe Cys Pro Val Ala Cys Cys Leu Asp
1               5                   10                  15

Pro Gly Gly Ala Ser Tyr Ser Cys Cys Arg Pro Leu Leu Asp Lys Trp
            20                  25                  30

Pro Thr Thr Leu Ser Arg His Leu Gly Gly Pro Cys Gln Val Asp Ala
        35                  40                  45

His Cys Ser Ala Gly His Ser Cys Ile Phe Thr Val Ser Gly Thr Ser
    50                  55                  60

Ser Cys Cys Pro Phe Pro Glu Ala Val Ala Cys Gly Asp Gly His His
65                  70                  75                  80

Cys Cys Pro Arg Gly Phe His Cys Ser Ala Asp Gly Arg Ser Cys Phe
                85                  90                  95

Gln Arg Ser Gly Asn Asn Ser Val Gly Ala Ile Gln Cys Pro Asp Ser
            100                 105                 110

Gln Phe Glu Cys Pro Asp Phe Ser Thr Cys Cys Val Met Val Asp Gly
        115                 120                 125

Ser Trp Gly Cys Cys Pro Met Pro Gln Ala Ser Cys Cys Glu Asp Arg
    130                 135                 140

Val His Cys Cys Pro His Gly Ala Phe Cys Asp Leu Val His Thr Arg
145                 150                 155                 160

Cys Ile Thr Pro Thr Gly Thr His Pro Leu Ala Lys Lys Leu Pro Ala
                165                 170                 175

Gln Arg Thr Asn Arg Ala Val Ala Leu Ser Ser Ser Val Met Cys Pro
            180                 185                 190

Asp Ala Arg Ser Arg Cys Pro Asp Gly Ser Thr Cys Cys Glu Leu Pro
        195                 200                 205

Ser Gly Lys Tyr Gly Cys Cys Pro Met Pro Asn Ala Thr Cys Cys Ser
    210                 215                 220
Asp His Leu His Cys Cys Pro Gln Asp Thr Val Cys Asp Leu Ile Gln
225                 230                 235                 240
Ser Lys Cys Leu Ser Lys Glu Asn Ala Thr Thr Asp Leu Leu Thr Lys
                245                 250                 255
Leu Pro Ala His Thr Val Gly Asp Val Lys Cys Asp Met Glu Val Ser
            260                 265                 270
Cys Pro Asp Gly Tyr Thr Cys Cys Arg Leu Gln Ser Gly Ala Trp Gly
        275                 280                 285
Cys Cys Pro Phe Thr Gln Ala Val Cys Cys Glu Asp His Ile His Cys
    290                 295                 300
Cys Pro Ala Gly Phe Thr Cys Asp Thr Gln Lys Gly Thr Cys Glu Gln
305                 310                 315                 320
Gly Pro His Gln Val Pro Trp Met Glu Lys Ala Pro Ala His Leu Ser
                325                 330                 335

Leu Pro Asp Pro Gln Ala Leu Lys Arg Asp Val Pro Cys Asp Asn Val
            340                 345                 350

Ser Ser Cys Pro Ser Ser Asp Thr Cys Cys Gln Leu Thr Ser Gly Glu
        355                 360                 365

Trp Gly Cys Cys Pro Ile Pro Glu Ala Val Cys Cys Ser Asp His Gln
    370                 375                 380

His Cys Cys Pro Gln Gly Tyr Thr Cys Val Ala Glu Gly Gln Cys Gln
385                 390                 395                 400

Arg Gly Ser Glu Ile Val Ala Gly Leu Glu Lys Met Pro Ala Arg Arg
                405                 410                 415

Ala Ser Leu Ser His Pro Arg Asp Ile Gly Cys Asp Gln His Thr Ser
            420                 425                 430

Cys Pro Val Gly Gln Thr Cys Cys Pro Ser Leu Gly Gly Ser Trp Ala
        435                 440                 445

Cys Cys Gln Leu Pro His Ala Val Cys Cys Glu Asp Arg Gln His Cys

450                 455                 460

Cys Pro Ala Gly Tyr Thr Cys Asn Val Lys Ala Arg Ser Cys Glu Lys
465                 470                 475                 480

Glu Val Val Ser Ala Gln Pro Ala Thr Phe Leu Ala Arg Ser Pro His
                485                 490                 495

Val Gly Val Lys Asp Val Glu Cys Gly Glu Gly His Phe Cys His Asp
            500                 505                 510

Asn Gln Thr Cys Cys Arg Asp Asn Arg Gln Gly Trp Ala Cys Cys Pro
        515                 520                 525

Tyr Arg Gln Gly Val Cys Cys Ala Asp Arg Arg His Cys Cys Pro Ala
    530                 535                 540

Gly Phe Arg Cys Ala Ala Arg Gly Thr Lys Cys Leu Arg Arg Glu Ala
545                 550                 555                 560

Pro Arg Trp Asp Ala Pro Leu Arg Asp Pro Ala Leu Arg Gln Leu Leu
                565                 570                 575

<210>  5

<211>  424

<212>  PRT

<213>  Homo sapiens


<400>  5

His Pro Asp Arg Ile Ile Phe Pro Asn His Ala Cys Glu Asp Pro Pro
1               5                   10                  15

Ala Val Leu Leu Glu Val Gln Gly Thr Leu Gln Arg Pro Leu Val Arg
            20                  25                  30

Asp Ser Arg Thr Ser Pro Ala Asn Cys Thr Trp Leu Ile Leu Gly Ser
        35                  40                  45

Lys Glu Gln Thr Val Thr Ile Arg Phe Gln Lys Leu His Leu Ala Cys
    50                  55                  60

Gly Ser Glu Arg Leu Thr Leu Arg Ser Pro Leu Gln Pro Leu Ile Ser
65                  70                  75                  80

Leu Cys Glu Ala Pro Pro Ser Pro Leu Gln Leu Pro Gly Gly Asn Val
                85                  90                  95

Thr Ile Thr Tyr Ser Tyr Ala Gly Ala Arg Ala Pro Met Gly Gln Gly
            100                 105                 110

Phe Leu Leu Ser Tyr Ser Gln Asp Trp Leu Met Cys Leu Gln Glu Glu
        115                 120                 125

Phe Gln Cys Leu Asn His Arg Cys Val Ser Ala Val Gln Arg Cys Asp
    130                 135                 140

Gly Val Asp Ala Cys Gly Asp Gly Ser Asp Glu Ala Gly Cys Ser Ser
145                 150                 155                 160

Asp Pro Phe Pro Gly Leu Thr Pro Arg Pro Val Pro Ser Leu Pro Cys
                165                 170                 175

Asn Val Thr Leu Glu Asp Phe Tyr Gly Val Phe Ser Ser Pro Gly Tyr
            180                 185                 190

Thr His Leu Ala Ser Val Ser His Pro Gln Ser Cys His Trp Leu Leu
        195                 200                 205

Asp Pro His Asp Gly Arg Arg Leu Ala Val Arg Phe Thr Ala Leu Asp
    210                 215                 220

Leu Gly Phe Gly Asp Ala Val His Val Tyr Asp Gly Pro Gly Pro Pro
225                 230                 235                 240

Glu Ser Ser Arg Leu Leu Arg Ser Leu Thr His Phe Ser Asn Gly Lys
                245                 250                 255

Ala Val Thr Val Glu Thr Leu Ser Gly Gln Ala Val Val Ser Tyr His
            260                 265                 270

Thr Val Ala Trp Ser Asn Gly Arg Gly Phe Asn Ala Thr Tyr His Val
        275                 280                 285

Arg Gly Tyr Cys Leu Pro Trp Asp Arg Pro Cys Gly Leu Gly Ser Gly
    290                 295                 300

Leu Gly Ala Gly Glu Gly Leu Gly Glu Arg Cys Tyr Ser Glu Ala Gln
305                 310                 315                 320

Arg Cys Asp Gly Ser Trp Asp Cys Ala Asp Gly Thr Asp Glu Glu Asp
                325                 330                 335

Cys Pro Gly Cys Pro Pro Gly His Phe Pro Cys Gly Ala Ala Gly Thr
            340                 345                 350

Ser Gly Ala Thr Ala Cys Tyr Leu Pro Ala Asp Arg Cys Asn Tyr Gln
        355                 360                 365

Thr Phe Cys Ala Asp Gly Ala Asp Glu Arg Arg Cys Arg His Cys Gln
    370                 375                 380

Pro Gly Asn Phe Arg Cys Arg Asp Glu Lys Cys Val Tyr Glu Thr Trp
385                 390                 395                 400

Val Cys Asp Gly Gln Pro Asp Cys Ala Asp Gly Ser Asp Glu Trp Asp
                405                 410                 415

Cys Ser Tyr Val Leu Pro Arg Lys
            420

<210> 6
<211> 121
<212> PRT
<213> Homo sapiens

<400> 6

Trp Ser Pro Gln Glu Glu Asp Arg Ile Ile Glu Gly Gly Ile Tyr Asp
1               5                   10                  15

Ala Asp Leu Asn Asp Glu Arg Val Gln Arg Ala Leu His Phe Val Ile
            20                  25                  30

Ser Glu Tyr Asn Lys Ala Thr Glu Asp Glu Tyr Tyr Arg Arg Leu Leu
        35                  40                  45

Arg Val Leu Arg Ala Arg Glu Gln Ile Val Gly Gly Val Asn Tyr Phe
    50                  55                  60

Phe Asp Ile Glu Val Gly Arg Thr Ile Cys Thr Lys Ser Gln Pro Asn
65                  70                  75                  80

Leu Asp Thr Cys Ala Phe His Glu Gln Pro Glu Leu Gln Lys Lys Gln
                85                  90                  95

Leu Cys Ser Phe Gln Ile Tyr Glu Val Pro Trp Glu Asp Arg Met Ser
            100                 105                 110

Leu Val Asn Ser Arg Cys Gln Glu Ala
        115                 120

<210>  7

<211>  394

<212>  PRT

<213>  Homo sapiens

<400>  7

Glu Asp Pro Gln Gly Asp Ala Ala Gln Lys Thr Asp Thr Ser His His
1               5                   10                  15

Asp Gln Asp His Pro Thr Phe Asn Lys Ile Thr Pro Asn Leu Ala Glu
            20                  25                  30

Phe Ala Phe Ser Leu Tyr Arg Gln Leu Ala His Gln Ser Asn Ser Thr
        35                  40                  45

Asn Ile Phe Phe Ser Pro Val Ser Ile Ala Thr Ala Phe Ala Met Leu
    50                  55                  60

Ser Leu Gly Thr Lys Ala Asp Thr His Asp Glu Ile Leu Glu Gly Leu
65                  70                  75                  80

Asn Phe Asn Leu Thr Glu Ile Pro Glu Ala Gln Ile His Glu Gly Phe
                85                  90                  95

Gln Glu Leu Leu Arg Thr Leu Asn Gln Pro Asp Ser Gln Leu Gln Leu
            100                 105                 110

Thr Thr Gly Asn Gly Leu Phe Leu Ser Glu Gly Leu Lys Leu Val Asp
        115                 120                 125

Lys Phe Leu Glu Asp Val Lys Lys Leu Tyr His Ser Glu Ala Phe Thr
    130                 135                 140

Val Asn Phe Gly Asp Thr Glu Glu Ala Lys Lys Gln Ile Asn Asp Tyr
145                 150                 155                 160

Val Glu Lys Gly Thr Gln Gly Lys Ile Val Asp Leu Val Lys Glu Leu
                165                 170                 175

Asp Arg Asp Thr Val Phe Ala Leu Val Asn Tyr Ile Phe Phe Lys Gly
            180                 185                 190

Lys Trp Glu Arg Pro Phe Glu Val Lys Asp Thr Glu Glu Glu Asp Phe
        195                 200                 205

His Val Asp Gln Val Thr Thr Val Lys Val Pro Met Met Lys Arg Leu
    210                 215                 220

Gly Met Phe Asn Ile Gln His Cys Lys Lys Leu Ser Ser Trp Val Leu
225                 230                 235                 240

Leu Met Lys Tyr Leu Gly Asn Ala Thr Ala Ile Phe Phe Leu Pro Asp
                245                 250                 255

Glu Gly Lys Leu Gln His Leu Glu Asn Glu Leu Thr His Asp Ile Ile
            260                 265                 270

Thr Lys Phe Leu Glu Asn Glu Asp Arg Arg Ser Ala Ser Leu His Leu
        275                 280                 285

Pro Lys Leu Ser Ile Thr Gly Thr Tyr Asp Leu Lys Ser Val Leu Gly
    290                 295                 300

Gln Leu Gly Ile Thr Lys Val Phe Ser Asn Gly Ala Asp Leu Ser Gly
305                 310                 315                 320

Val Thr Glu Glu Ala Pro Leu Lys Leu Ser Lys Ala Val His Lys Ala
                325                 330                 335

Val Leu Thr Ile Asp Glu Lys Gly Thr Glu Ala Ala Gly Ala Met Phe
            340                 345                 350
Leu Glu Ala Ile Pro Met Ser Ile Pro Pro Glu Val Lys Phe Asn Lys
        355                 360                 365
Pro Phe Val Phe Leu Met Ile Glu Gln Asn Thr Lys Ser Pro Leu Phe
    370                 375                 380
Met Gly Lys Val Val Asn Pro Thr Gln Lys
385                 390
<210> 8
<211> 250
<212> PRT
<213> Homo sapiens
<400> 8

<210>  9
<211>  238
<212>  PRT

<213>  Homo sapiens

<400>  9

Gln Val Ser Ala Thr Ala Ser Pro Ser Gly Ser Leu Gly Ala Pro Asp
1               5                   10                  15

Cys Pro Glu Val Cys Thr Cys Val Pro Gly Gly Leu Ala Ser Cys Ser
            20                  25                  30

Ala Leu Ser Leu Pro Ala Val Pro Pro Gly Leu Ser Leu Arg Leu Arg
        35                  40                  45

Ala Leu Leu Leu Asp His Asn Arg Val Arg Ala Leu Pro Pro Gly Ala
    50                  55                  60

Phe Ala Gly Ala Gly Ala Leu Gln Arg Leu Asp Leu Arg Glu Asn Gly
65                  70                  75                  80

Leu His Ser Val His Val Arg Ala Phe Trp Gly Leu Gly Ala Leu Gln
                85                  90                  95

Leu Leu Asp Leu Ser Ala Asn Gln Leu Glu Ala Leu Ala Pro Gly Thr
            100                 105                 110

Phe Ala Pro Leu Arg Ala Leu Arg Asn Leu Ser Leu Ala Gly Asn Arg
        115                 120                 125

Leu Ala Arg Leu Glu Pro Ala Ala Leu Gly Ala Leu Pro Leu Leu Arg
    130                 135                 140

Ser Leu Ser Leu Gln Asp Asn Glu Leu Ala Ala Leu Ala Pro Gly Leu
145                 150                 155                 160

Leu Gly Arg Leu Pro Ala Leu Asp Ala Leu His Leu Arg Gly Asn Pro
                165                 170                 175

Trp Gly Cys Gly Cys Ala Leu Arg Pro Leu Cys Ala Trp Leu Arg Arg
            180                 185                 190

His Pro Leu Pro Ala Ser Glu Ala Glu Thr Val Leu Cys Val Trp Pro
        195                 200                 205

Gly Arg Leu Thr Leu Ser Pro Leu Thr Ala Phe Ser Asp Ala Ala Phe
    210                 215                 220

Ser His Cys Ala Gln Pro Leu Ala Leu Arg Asp Leu Ala Val

225                 230                 235

<210>  10

<211>  118

<212>  PRT

<213>  Homo sapiens

<400>  10

Leu Arg Val Arg Gly Glu Val Ala Pro Asp Ala Lys Ser Phe Val Leu

1               5                   10                  15

Asn Leu Gly Lys Asp Ser Asn Asn Leu Cys Leu His Phe Asn Pro Arg

            20                  25                  30

Phe Asn Ala His Gly Asp Ala Asn Thr Ile Val Cys Asn Ser Lys Asp

        35                  40                  45

Gly Gly Ala Trp Gly Thr Glu Gln Arg Glu Ala Val Phe Pro Phe Gln

    50                  55                  60

Pro Gly Ser Val Ala Glu Val Cys Ile Thr Phe Asp Gln Ala Asn Leu

65                  70                  75                  80

Thr Val Lys Leu Pro Asp Gly Tyr Glu Phe Lys Phe Pro Asn Arg Leu

85                  90                  95

Asn Leu Glu Ala Ile Asn Tyr Met Ala Ala Asp Gly Asp Phe Lys Ile
            100                 105                 110

Lys Cys Val Ala Phe Asp
        115

<210>  11

<211>  145

<212>  PRT

<213>  Homo sapiens

<400>  11

Ala Pro Lys Pro Ala Thr Val Val Thr Gly Ser Gly His Ala Ser Ser
1               5                   10                  15

Thr Pro Gly Gly Glu Lys Glu Thr Ser Ala Thr Gln Arg Ser Ser Val
            20                  25                  30

Pro Ser Ser Thr Glu Lys Asn Ala Phe Asn Ser Ser Leu Glu Asp Pro
        35                  40                  45

Ser Thr Asp Tyr Tyr Gln Glu Leu Gln Arg Asp Ile Ser Glu Met Phe
    50                  55                  60
Leu Gln Ile Tyr Lys Gln Gly Gly Phe Leu Gly Leu Ser Asn Ile Lys
65                  70                  75                  80
Phe Arg Pro Gly Ser Val Val Val Gln Leu Thr Leu Ala Phe Arg Glu
                85                  90                  95
Gly Thr Ile Asn Val His Asp Val Glu Thr Gln Phe Asn Gln Tyr Lys
            100                 105                 110
Thr Glu Ala Ala Ser Arg Tyr Asn Leu Thr Ile Ser Asp Val Ser Val
        115                 120                 125
Ser Asp Val Pro Phe Pro Phe Ser Ala Gln Ser Gly Ala Gly Val Pro
    130                 135                 140
Gly
145
<210> 12
<211> 636
<212> PRT

<213>  Homo sapiens

<400>  12

Glu Glu Glu Asp Lys Lys Glu Asp Val Gly Thr Val Val Gly Ile Asp
1               5                   10                  15

Leu Gly Thr Thr Tyr Ser Cys Val Gly Val Phe Lys Asn Gly Arg Val
            20                  25                  30

Glu Ile Ile Ala Asn Asp Gln Gly Asn Arg Ile Thr Pro Ser Tyr Val
        35                  40                  45

Ala Phe Thr Pro Glu Gly Glu Arg Leu Ile Gly Asp Ala Ala Lys Asn
    50                  55                  60

Gln Leu Thr Ser Asn Pro Glu Asn Thr Val Phe Asp Ala Lys Arg Leu
65                  70                  75                  80

Ile Gly Arg Thr Trp Asn Asp Pro Ser Val Gln Gln Asp Ile Lys Phe
                85                  90                  95

Leu Pro Phe Lys Val Val Glu Lys Lys Thr Lys Pro Tyr Ile Gln Val
            100                 105                 110

Asp Ile Gly Gly Gly Gln Thr Lys Thr Phe Ala Pro Glu Glu Ile Ser
        115                 120                 125

Ala Met Val Leu Thr Lys Met Lys Glu Thr Ala Glu Ala Tyr Leu Gly
    130                 135                 140

Lys Lys Val Thr His Ala Val Val Thr Val Pro Ala Tyr Phe Asn Asp
145                 150                 155                 160

Ala Gln Arg Gln Ala Thr Lys Asp Ala Gly Thr Ile Ala Gly Leu Asn
                165                 170                 175

Val Met Arg Ile Ile Asn Glu Pro Thr Ala Ala Ala Ile Ala Tyr Gly
            180                 185                 190

Leu Asp Lys Arg Glu Gly Glu Lys Asn Ile Leu Val Phe Asp Leu Gly
        195                 200                 205

Gly Gly Thr Phe Asp Val Ser Leu Leu Thr Ile Asp Asn Gly Val Phe
    210                 215                 220

Glu Val Val Ala Thr Asn Gly Asp Thr His Leu Gly Gly Glu Asp Phe

225                 230                 235                 240

Asp Gln Arg Val Met Glu His Phe Ile Lys Leu Tyr Lys Lys Lys Thr

                245                 250                 255

Gly Lys Asp Val Arg Lys Asp Asn Arg Ala Val Gln Lys Leu Arg Arg

            260                 265                 270

Glu Val Glu Lys Ala Lys Arg Ala Leu Ser Ser Gln His Gln Ala Arg

        275                 280                 285

Ile Glu Ile Glu Ser Phe Tyr Glu Gly Glu Asp Phe Ser Glu Thr Leu

    290                 295                 300

Thr Arg Ala Lys Phe Glu Glu Leu Asn Met Asp Leu Phe Arg Ser Thr

305                 310                 315                 320

Met Lys Pro Val Gln Lys Val Leu Glu Asp Ser Asp Leu Lys Lys Ser

                325                 330                 335

Asp Ile Asp Glu Ile Val Leu Val Gly Gly Ser Thr Arg Ile Pro Lys

            340                 345                 350

Ile Gln Gln Leu Val Lys Glu Phe Phe Asn Gly Lys Glu Pro Ser Arg
        355                 360                 365

Gly Ile Asn Pro Asp Glu Ala Val Ala Tyr Gly Ala Ala Val Gln Ala
    370                 375                 380

Gly Val Leu Ser Gly Asp Gln Asp Thr Gly Asp Leu Val Leu Leu Asp
385                 390                 395                 400

Val Cys Pro Leu Thr Leu Gly Ile Glu Thr Val Gly Gly Val Met Thr
                405                 410                 415

Lys Leu Ile Pro Arg Asn Thr Val Val Pro Thr Lys Lys Ser Gln Ile
            420                 425                 430

Phe Ser Thr Ala Ser Asp Asn Gln Pro Thr Val Thr Ile Lys Val Tyr
        435                 440                 445

Glu Gly Glu Arg Pro Leu Thr Lys Asp Asn His Leu Leu Gly Thr Phe
    450                 455                 460

Asp Leu Thr Gly Ile Pro Pro Ala Pro Arg Gly Val Pro Gln Ile Glu
465                 470                 475                 480

Val Thr Phe Glu Ile Asp Val Asn Gly Ile Leu Arg Val Thr Ala Glu
                485                 490                 495

Asp Lys Gly Thr Gly Asn Lys Asn Lys Ile Thr Ile Thr Asn Asp Gln
            500                 505                 510

Asn Arg Leu Thr Pro Glu Glu Ile Glu Arg Met Val Asn Asp Ala Glu
        515                 520                 525

Lys Phe Ala Glu Glu Asp Lys Lys Leu Lys Glu Arg Ile Asp Thr Arg
    530                 535                 540

Asn Glu Leu Glu Ser Tyr Ala Tyr Ser Leu Lys Asn Gln Ile Gly Asp
545                 550                 555                 560

Lys Glu Lys Leu Gly Gly Lys Leu Ser Ser Glu Asp Lys Glu Thr Met
                565                 570                 575

Glu Lys Ala Val Glu Glu Lys Ile Glu Trp Leu Glu Ser His Gln Asp
            580                 585                 590

Ala Asp Ile Glu Asp Phe Lys Ala Lys Lys Lys Glu Leu Glu Glu Ile
        595                 600                 605
Val Gln Pro Ile Ile Ser Lys Leu Tyr Gly Ser Ala Gly Pro Pro Pro
    610                 615                 620
Thr Gly Glu Glu Asp Thr Ala Glu Lys Asp Glu Leu
625                 630                 635

## Description

### Cross Reference

This application claims priority to U.S. Provisional Patent Application Serial No. 62/266189 filed December 11, 2015, incorporated by reference herein in its entirety.

### Federal Funding Statement:

This invention was made with government support under Grant No. P20GM103548 awarded by the National Institutes of Health. The government has certain rights in the invention.

### Background

The 5-year survival rate for women diagnosed with local breast cancer (BCa) is 98.6%. Survival declines to 83.8% for regional stage and plummets to 23.3% for distant stage [3]. Only 5% of U.S. women present with metastatic BCa when first diagnosed [4]; however, most BCa-related deaths are due to incurable metastatic disease [5], not the primary diagnosis. Unfortunately, recurrence of BCa is most often found when patients report symptoms, such as shortness of breath, chronic cough, weight loss or bone pain.

### Summary of the Invention

In one aspect, the invention provides compositions comprising at least 2 antibody detection markers, wherein the antibody detection markers comprise reagents for detecting human autoantibodies against at least two proteins selected from the group consisting of A1AT (Alpha-1 antitrypsin) (SEQ ID NO:7), ANGPTL4 (Angiopoietin-like 4) (SEQ ID NO:1), LRP10 (LDL Receptor Related Protein 10) (SEQ ID NO:5), GFRA1 (GDNF Family Receptor Alpha 1) (SEQ ID NO:3), LGALS3 (Galectin-3) (SEQ ID NO:8), CST2 (Cystatin SA) (SEQ ID NO:6), DKK1 (Dickkopf WNT Signaling Pathway Inhibitor 1) (SEQ ID NO:2), CAPC (Cytokeratin-Associated Protein In Cancer) (SEQ ID NO:9), GRP78 (78 kDa glucose-regulated protein) (SEQ ID NO: 12) and GRN (Granulin) (SEQ ID NO:4), wherein at least one of the proteins is selected from the group consisting of A1AT, LGALS3, and CAPC. In one embodiment, the composition includes reagents for detecting human autoantibodies against at least 3 proteins selected from the group consisting of A1AT, ANGPTL4, LRP10, GFRA1, LGALS3, CST2, DKK1, CAPC, GRP78, and GRN. In another embodiment, the composition includes reagents for detecting human autoantibodies against at least 5, 6, 7, 8, or all 9 proteins in the recited group. In further embodiments, the composition consists of between 2 and 1000 antibody detection markers, or between 4 and 500 antibody detection markers. In another embodiment, the composition includes reagents for detecting human autoantibodies against at least 2, 3, 4, or all 5 of ANGPTL4, GFRA1, LGALS3, DKK1 and GRN. In a further embodiment, the composition further comprises reagents for detecting human autoantibodies against one or both of GAL1 and MUC1. In one embodiment, the reagents for detecting human autoantibodies comprise the at least two proteins, or antigenic fragments thereof. In another embodiment, the at least two proteins, or antigenic fragments thereof comprise native extracellular domains and/or native secreted proteins or antigenic fragments thereof. In a further embodiment, the reagents are detectably labeled. In a still further embodiment, the at least two proteins, or antigenic fragments thereof, are expressed as a fusion with a detectable domain, including but not limited to an Fc domain. In another embodiment, the reagents are immobilized on a surface of a solid support.

In another aspect, the invention provides methods for monitoring breast cancer therapy, comprising
(a) contacting a bodily fluid sample from a subject who is undergoing or has undergone breast cancer therapy with one or more reagents for detecting autoantibodies against one or more proteins selected from the group consisting of A1AT, ANGPTL4, LRP10, GFRA1, LGALS3, CST2, DKK1, CAPC, GRP78, and GRN; and
(b) determining an amount of autoantibodies against the one or more proteins in the bodily fluid sample;
wherein a decrease in the amount of autoantibodies relative to a control, such as a baseline level of autoantibodies in a similar bodily fluid sample from the subject indicates efficacy of the breast cancer therapy in the subject.

In another aspect, the invention provides methods for prognosing breast cancer recurrence, comprising
(a) contacting a bodily fluid sample from a subject who has received breast cancer therapy with one or more reagents for detecting autoantibodies against one or more proteins selected from the group consisting of A1AT, ANGPTL4, LRP10, GFRA1, LGALS3, CST2, DKK1, CAPC, GRP78, and GRN; and
(b) determining an amount of autoantibodies against the one or more proteins in the bodily fluid sample;
wherein an increase in the amount of autoantibodies relative to a baseline level of autoantibodies in a control, such as a similar bodily fluid sample from the subject indicates a likelihood of breast cancer recurrence in the subject.

In various embodiments, the reagents comprise reagents for detecting autoantibodies 3, 4, 5, 6, 7, 8, or all 9 of the recited proteins. In another embodiment, the reagents comprise the composition of any embodiment or combination of embodiments of the invention.

In one embodiment, the one or more reagents comprise 2, 3, 4, or all 5 proteins, or antigenic fragments thereof, selected from the group consisting of ANGPTL4, GFRA1, LGALS3, DKK1 and GRN.

In another aspect, the invention provides methods for monitoring breast cancer therapy, comprising
(a) contacting a bodily fluid sample from a subject who is undergoing or has undergone breast cancer therapy with one or more reagents for detecting autoantibodies against GRP78; and
(b) determining an amount of autoantibodies against GRP78 in the bodily fluid sample;
wherein an increase in the amount of autoantibodies against GRP78 relative to a control, such as a baseline level of autoantibodies in a similar bodily fluid sample from the subject indicates efficacy of the breast cancer therapy in the subject.

In a further aspect, the invention provides methods for prognosing breast cancer recurrence, comprising
(a) contacting a bodily fluid sample from a subject who has received breast cancer therapy with one or more reagents for detecting autoantibodies against GRP78; and
(b) determining an amount of autoantibodies against GRP78 in the bodily fluid sample;
wherein a decrease in the amount of autoantibodies relative to a baseline level of autoantibodies against GRP78 in a control, such as a similar bodily fluid sample from the subject indicates a likelihood of breast cancer recurrence in the subject. In another embodiment, the contacting comprises use of ELISA. In a further embodiment, the bodily fluid sample comprises a serum sample from the subject. In one embodiment, the subject has had surgery to remove the primary tumor prior to carrying out the method of the invention. In another embodiment, the subject is receiving or has received radiation therapy and chemotherapy, or hormonal therapy and chemotherapy. In a further embodiment, the contacting comprises use of Longitudinal Assay Screening, wherein all target biomarkers may be detected and quantitated within a single test and dilution. In a still further embodiment, the bodily fluid sample comprises a blood sample from the subject. In one embodiment, if no decrease is determined in the amount of autoantibodies relative to a baseline level of autoantibodies in a similar bodily fluid sample from the subject, the method further comprises altering the breast cancer therapy being administered to the subject. In another embodiment, the method is used to detect breast cancer recurrence.

### Brief Description of the Figures

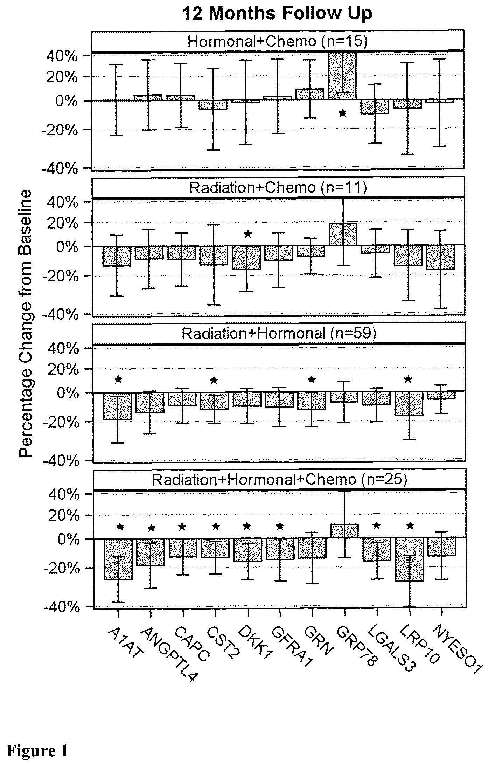

**Figure 1****.** Observed geometric mean changes (with 95% confidence intervals) from baseline for autoantibodies levels against 11 tumor-associated antigens according to treatment regimen after 12 months follow-up. * indicates p-value < 0.05. There were no significant changes observed for surgery only or individual therapies (i.e. hormonal, radiation or chemotherapy).

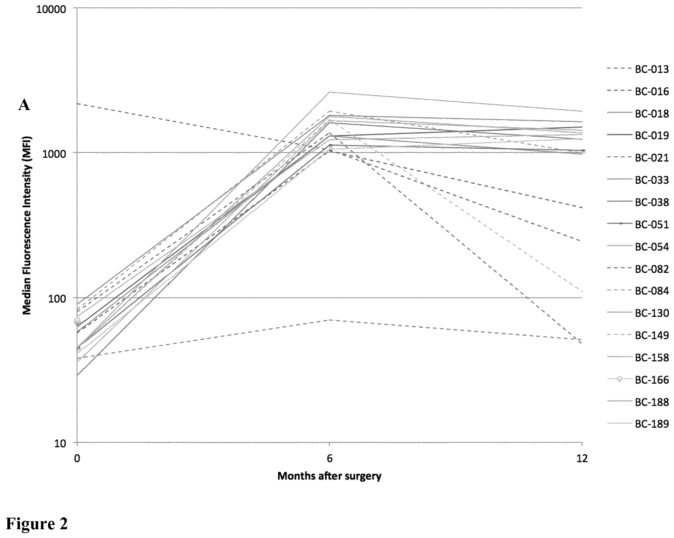

**Figure 2****.** Antibody response against ERBB2-rFc in patients diagnosed with HER2 positive breast cancer treated with HERCEPTIN®. Three longitudinal blood draws were collected, immediately before surgery, 6 months and 12 months after surgery. The solid lines indicate patients who were still receiving HERCEPTIN® therapy at their 12-month visit, and the dashed lines represent patients that discontinued HERCEPTIN® therapy prior to their 12-month visit. A light, green circle represents patient BC-166 because only the baseline blood draw was acquired. Geometric mean values were 48, 1206 and 600 over time. Follow up levels were significantly greater than baseline (p<0.0001), but 6 and 12-month levels were not different (p=0.09).

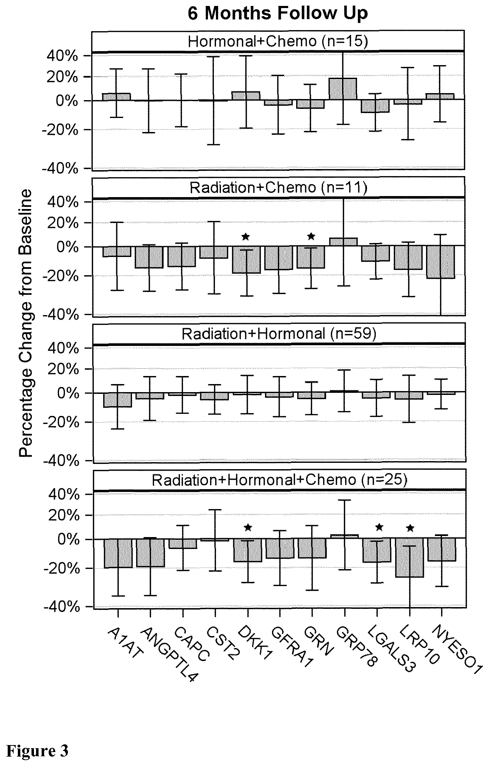

**Figure 3****.** Observed geometric mean changes (with 95% confidence intervals) from baseline for autoantibodies levels against 11 tumor-associated antigens according to treatment regimen after 6 months follow-up. * indicates p-value < 0.05. There were no significant changes observed for surgery only or individual therapies (i.e. hormonal, radiation, or chemotherapy).

### Detailed Description of the Invention

All references cited are herein incorporated by reference in their entirety.

Within this application, unless otherwise stated, the techniques utilized may be found in any of several well-known references such as: Molecular Cloning: A Laboratory Manual (Sambrook, et al., 1989, Cold Spring Harbor Laboratory Press), Gene Expression Technology (Methods in Enzymology, Vol. 185, edited by D. Goeddel, 1991. Academic Press, San Diego, CA), "Guide to Protein Purification" in Methods in Enzymology (M.P. Deutshcer, ed., (1990) Academic Press, Inc.); PCR Protocols: A Guide to Methods and Applications (Innis, et al. 1990. Academic Press, San Diego, CA), Culture of Animal Cells: A Manual of Basic Technique, 2nd Ed. (R.I. Freshney. 1987. Liss, Inc. New York, NY), Gene Transfer and Expression Protocols, pp. 109-128, ed. E.J. Murray, The Humana Press Inc., Clifton, N.J.), and the Ambion 1998 Catalog (Ambion, Austin, TX).

In a first aspect, the present invention provides compositions comprising at least 2 antibody detection markers, wherein the antibody detection markers comprise reagents for detecting human autoantibodies against at least two proteins selected from the group consisting of A1AT (Alpha-1 antitrypsin) (SEQ ID NO:7), ANGPTL4 (Angiopoietin-like 4) (SEQ ID NO:1), LRP10 (LDL Receptor Related Protein 10) (SEQ ID NO:5), GFRA1 (GDNF Family Receptor Alpha 1) (SEQ ID NO:3), LGALS3 (Galectin-3) (SEQ ID NO:8), CST2 (Cystatin SA) (SEQ ID NO:6), DKK1 (Dickkopf WNT Signaling Pathway Inhibitor 1) (SEQ ID NO:2), CAPC (Cytokeratin-Associated Protein In Cancer) (SEQ ID NO:9), GRP78 (78 kDa glucose-regulated protein) (SEQ ID NO: 12) and GRN (Granulin) (SEQ ID NO:4), wherein at least one of the proteins is selected from the group consisting of A1AT, LGALS3, and CAPC.

The inventors have unexpectedly discovered that autoantibodies against the recited proteins provide an indication of efficacy of therapy for a subject being treated for BCa, and/or BCa recurrence. Thus, the compositions of the invention can be used, for example, in diagnostic assays to monitor efficacy of breast cancer therapy, and/or recurrence. In one embodiment, the composition includes reagents for detecting human autoantibodies against at least two proteins selected from the group consisting of A1AT, ANGPTL4, LRP10, GFRA1, LGALS3, CST2, DKK1, CAPC, GRP78, and GRN.

In various embodiments, the composition comprises or consists of reagents for detecting human autoantibodies against at least three, four, five, six, seven, eight, or all nine proteins in the recited group. In various further embodiments, the composition comprises or consists of reagents for detecting human autoantibodies against 2, 3, 4, or all 5 of ANGPTL4, GFRA1, LGALS3, DKK1 and GRN.

In a further embodiment, the composition further comprises reagents for detecting human autoantibodies against one or both of GAL1 and MUC1.

In various further embodiments, the composition comprises or consists of between 2-1000, 3-1000, 4-1000, 5-1000, 6-1000, 7-1000, 8-1000, 9-1000, 2-500, 3-500, 4-500, 5-500, 6-500, 7-500, 8-500, 9-500, 2-100, 3-100, 4-100, 5-100, 6-100, 7-100, 8-100, 9-100, 2-50, 3-50, 4-50, 5-50, 6-50, 7-50, 8-50, 9-50, 2-25, 3-25, 4-25, 5-25, 6-25, 7-25, 8-25, or 9-25 antibody detection reagents.

As will be understood by those of skill in the art, the compositions may include additional antibody detection markers and controls as is appropriate for an intended use of the composition.

The antibody detection markers may be any suitable reagents that can be used to detect antibodies against the recited proteins, including but not limited to the recited protein, a secreted version of the protein (such as a native secreted form of the protein), or an extracellular domain of the protein. Secreted proteins are more easily delivered from tumor cells to lymph nodes, where interactions of immune cells take place resulting in abundant high-affinity antibodies. Membrane surface proteins are commonly released in a soluble form from tumor cells through metalloproteinase-dependent cleavage. The shed proteins are more easily transferred to the lymph nodes than intracellular protein. Thus, in one embodiment the antibody detection marker can be a secreted or membrane portion of the recited protein. Exemplary amino acid sequences of the recited human proteins are shown below, as noted: Full length protein without signal sequence: ANGPTL4, A1AT, CST2, DKK1, GFRA1, GRN, GRP78, GAL1, MUC1.
Extracellular domain region without signal sequence: CAPC, LRP10
Full length (no signal sequence predicted in protein): LGALS3

**ANGTPL4** (SEQ ID NO:1) 
KSPRFASWDEMNVLAHGLLQLGQGLREHAERTRSQLSALERRLSACGSACQGTEGS
TDLPLAPESRVDPEVLHSLQTQLKAQNSRIQQLFHKVAQQQRHLEKQHLRIQHLQSQ
FGLLDHKHLDHEVAKPARRKRLPEMAQPVDPAHNVSRLHRLPRDCQELFQVGERQS
GLFEIQPQGSPPFLVNCKMTSDGGWTVIQRRHDGSVDFNRPWEAYKAGFGDPHGEF
WLGLEKVHSITGDRNSRLAVQLRDWDGNAELLQFSVHLGGEDTAYSLQLTAPVAG
QLGATTVPPSGLSVPFSTWDQDHDLRRDKNCAKSLSGGWWFGTCSHSNLNGQYFRS
IPQQRQKLKKGIFWKTWRGRYYPLQATTMLIQPMAAEAAS
**DKK1** (SEQ ID NO:2) 
VSATLNSVLNSNAIKNLPPPLGGAAGHPGSAVSAAPGILYPGGNKYQTIDNYQPYPC
AEDEECGTDEYCASPTRGGDAGVQICLACRKRRKRCMRHAMCCPGNYCKNGICVSS
DQNHFRGEIEETITESFGNDHSTLDGYSRRTTLSSKMYHTKGQEGSVCLRSSDCASGL
 
CCARHFWSKICKPVLKEGQVCTKHRRKGSHGLEIFQRCYCGEGLSCRIQKDHHQAS
NSSRLHTCQRH
**GFRA1** (SEQ ID NO:3) 
DRLDCVKASDQCLKEQSCSTKYRTLRQCVAGKETNFSLASGLEAKDECRSAMEALK
QKSLYNCRCKRGMKKEKNCLRIYWSMYQSLQGNDLLEDSPYEPVNSRLSDIFRVVP
FISDVFQQVEHIPKGNNCLDAAKACNLDDICKKYRSAYITPCTTSVSNDVCNRRKCH
KALRQFFDKVPAKHSYGMLFCSCRDIACTERRRQTIVPVCSYEEREKPNCLNLQDSC
KTNYICRSRLADFFTNCQPESRSVSSCLKENYADCLLAYSGLIGTVMTPNYIDSSSLSV
APWCDCSNSGNDLEECLKFLNFFKDNTCLKNAIQAFGNGSDVTVWQPAFPVQTTTA
TTTTALRVKNKPLGPAGSENEIPTHVLPPCANLQAQKLKSNVSGNTHLCISNGNYEK
EGLGASSHITTKSMAAPPSCGLSPLLVLVVTALSTLLSLTETS
**GRN** (SEQ ID NO:4) 
TRCPDGQFCPVACCLDPGGASYSCCRPLLDKWPTTLSRHLGGPCQVDAHCSAGHSCI
FTVSGTSSCCPFPEAVACGDGHHCCPRGFHCSADGRSCFQRSGNNSVGAIQCPDSQF
ECPDFSTCCVMVDGSWGCCPMPQASCCEDRVHCCPHGAFCDLVHTRCITPTGTHPL
AKKLPAQRTNRAVALSSSVMCPDARSRCPDGSTCCELPSGKYGCCPMPNATCCSDH
LHCCPQDTVCDLIQSKCLSKENATTDLLTKLPAHTVGDVKCDMEVSCPDGYTCCRL
QSGAWGCCPFTQAVCCEDHIHCCPAGFTCDTQKGTCEQGPHQVPWMEKAPAHLSLP
DPQALKRDVPCDNVSSCPSSDTCCQLTSGEWGCCPIPEAVCCSDHQHCCPQGYTCVA
EGQCQRGSEIVAGLEKMPARRASLSHPRDIGCDQHTSCPVGQTCCPSLGGSWACCQL
PHAVCCEDRQHCCPAGYTCNVKARSCEKEVVSAQPATFLARSPHVGVKDVECGEG
HFCHDNQTCCRDNRQGWACCPYRQGVCCADRRHCCPAGFRCAARGTKCLRREAPR
WDAPLRDPALRQLL
**LRP10** (SEQ ID NO:5) 
HPDRIIFPNHACEDPPAVLLEVQGTLQRPLVRDSRTSPANCTWLILGSKEQTVTIRFQK
LHLACGSERLTLRSPLQPLISLCEAPPSPLQLPGGNVTITYSYAGARAPMGQGFLLSYS
QDWLMCLQEEFQCLNHRCVSAVQRCDGVDACGDGSDEAGCSSDPFPGLTPRPVPSL
PCNVTLEDFYGVFSSPGYTHLASVSHPQSCHWLLDPHDGRRLAVRFTALDLGFGDA
VHVYDGPGPPESSRLLRSLTHFSNGKAVTVETLSGQAVVSYHTVAWSNGRGFNATY
HVRGYCLPWDRPCGLGSGLGAGEGLGERCYSEAQRCDGSWDCADGTDEEDCPGCP
 
PGHFPCGAAGTSGATACYLPADRCNYQTFCADGADERRCRHCQPGNFRCRDEKCV
YETWVCDGQPDCADGSDEWDCSYVLPRK
**CST2** (SEQ ID NO:6) 
WSPQEEDRIIEGGIYDADLNDERVQRALHFVISEYNKATEDEYYRRLLRVLRAREQIV
GGVNYFFDIEVGRTICTKSQPNLDTCAFHEQPELQKKQLCSFQIYEVPWEDRMSLVN
SRCQEA
**A1AT** (SEQ ID NO:7) 
EDPQGDAAQKTDTSHHDQDHPTFNKITPNLAEFAFSLYRQLAHQSNSTNIFFSPVSIA
TAFAMLSLGTKADTHDEILEGLNFNLTEIPEAQIHEGFQELLRTLNQPDSQLQLTTGN
GLFLSEGLKLVDKFLEDVKKLYHSEAFTVNFGDTEEAKKQINDYVEKGTQGKIVDLV
KELDRDTVFALVNYIFFKGKWERPFEVKDTEEEDFHVDQVTTVKVPMMKRLGMFNI
QHCKKLSSWVLLMKYLGNATAIFFLPDEGKLQHLENELTHDIITKFLENEDRRSASLH
LPKLSITGTYDLKSVLGQLGITKVFSNGADLSGVTEEAPLKLSKAVHKAVLTIDEKGT
EAAGAMFLEAIPMSIPPEVKFNKPFVFLMIEQNTKSPLFMGKVVNPTQK
**LGALS3** (SEQ ID NO:8)
**CAPC** (SEQ ID NO:9) 
QVSATASPSGSLGAPDCPEVCTCVPGGLASCSALSLPAVPPGLSLRLRALLLDHNRVR
ALPPGAFAGAGALQRLDLRENGLHSVHVRAFWGLGALQLLDLSANQLEALAPGTFA
PLRALRNLSLAGNRLARLEPAALGALPLLRSLSLQDNELAALAPGLLGRLPALDALH
LRGNPWGCGCALRPLCAWLRRHPLPASEAETVLCVWPGRLTLSPLTAFSDAAFSHC
AQPLALRDLAV
**GAL1** (SEQ ID NO:10) 
LRVRGEVAPDAKSFVLNLGKDSNNLCLHFNPRFNAHGDANTIVCNSKDGGAWGTE
QREAVFPFQPGSVAEVCITFDQANLTVKLPDGYEFKFPNRLNLEAINYMAADGDFKI
KCVAFD
**MUC1** (SEQ ID NO:11) 
APKPATVVTGSGHASSTPGGEKETSATQRSSVPSSTEKNAFNSSLEDPSTDYYQELQR
DISEMFLQIYKQGGFLGLSNIKFRPGSVVVQLTLAFREGTINVHDVETQFNQYKTEAA
SRYNLTISDVSVSDVPFPFSAQSGAGVPG
**GRP78** (SEQ ID NO:12)

EEEDKKEDVGTVVGIDLGTTYSCVGVFKNGRVEIIANDQGNRITPSYVAFTPEGERLI
GDAAKNQLTSNPENTVFDAKRLIGRTWNDPSVQQDIKFLPFKVVEKKTKPYIQVDIG
GGQTKTFAPEEISAMVLTKMKETAEAYLGKKVTHAVVTVPAYFNDAQRQATKDAG
TIAGLNVMRIINEPTAAAIAYGLDKREGEKNILVFDLGGGTFDVSLLTIDNGVFEVVA
TNGDTHLGGEDFDQRVMEHFIKLYKKKTGKDVRKDNRAVQKLRREVEKAKRALSS
QHQARIEIESFYEGEDFSETLTRAKFEELNMDLFRSTMKPVQKVLEDSDLKKSDIDEI
VLVGGSTRIPKIQQLVKEFFNGKEPSRGINPDEAVAYGAAVQAGVLSGDQDTGDLVL
LDVCPLTLGIETVGGVMTKLIPRNTVVPTKKSQIFSTASDNQPTVTIKVYEGERPLTK
DNHLLGTFDLTGIPPAPRGVPQIEVTFEIDVNGILRVTAEDKGTGNKNKITITNDQNRL
TPEEIERMVNDAEKFAEEDKKLKERIDTRNELESYAYSLKNQIGDKEKLGGKLSSED
KETMEKAVEEKIEWLESHQDADIEDFKAKKKELEEIVQPIISKLYGSAGPPPTGEEDT
AEKDEL

In a further embodiment, the antibody detection marker is a protein, such as those disclosed above, that is in its native form. As disclosed in the accompanying examples, the inventors utilized a eukaryotic expression system to generate conformation-carrying tumor antigens that are properly folded and contain non-continuous epitopes for use in the detection of autoantibodies. In another embodiment, the reagents comprise the at least two proteins, or antigenic fragments thereof, and wherein the at least two proteins, or antigenic fragments thereof, are expressed as a fusion with a detectable domain. The protein may be used in any suitable format; in one non-limiting embodiment, the protein may be a surface-bound Fc fusion protein, or secreted Fc fusion protein.

In all of the above embodiments, the antibody detection reagents can be labeled with a detectable label. In one embodiment, the detectable labels for reagents to detect autoantibodies against one protein are distinguishable from the detectable labels to detect autoantibodies against the other protein. Methods for detecting the label include, but are not limited to spectroscopic, photochemical, biochemical, immunochemical, physical or chemical techniques. Any suitable detectable label can be used.

The compositions can be stored frozen, in lyophilized form, or as a solution. In one embodiment, the compositions can be placed on a surface of a solid support. Any suitable solid support may be used. Examples of such supports include, but are not limited to, microarrays, beads, columns, optical fibers, wipes, nitrocellulose, nylon, glass, quartz, diazotized membranes (paper or nylon), silicones, polyformaldehyde, cellulose, cellulose acetate, paper, ceramics, metals, metalloids, semiconductive materials, coated beads, magnetic particles; plastics such as polyethylene, polypropylene, and polystyrene; and gel-forming materials, such as proteins (e.g., gelatins), lipopolysaccharides, silicates, agarose, polyacrylamides, methylmethracrylate polymers; sol gels; porous polymer hydrogels; nanostructured surfaces; nanotubes (such as carbon nanotubes), and nanoparticles (such as gold nanoparticles or quantum dots). This embodiment facilitates use of the compositions in various detection assays. For example, anti- IgG can be used to precoat the wells of a microwell plate and the antibody detection reagents (such as the proteins discussed herein) can be added to the precoated wells.

In a second aspect, the present invention provides methods for monitoring breast cancer therapy, comprising
(a) contacting a bodily fluid sample from a subject who is undergoing or has undergone breast cancer therapy with one or more reagents for detecting autoantibodies against one or more proteins selected from the group consisting of A1AT, ANGPTL4, LRP10, , GFRA1, LGALS3, CST2, DKK1, CAPC, GRP78, and GRN; and
(b) determining an amount of autoantibodies against the one or more proteins in the bodily fluid sample;
wherein a decrease in the amount of autoantibodies relative to a baseline level of autoantibodies in a control, such as a similar bodily fluid sample from the subject indicates efficacy of the breast cancer therapy in the subject.

In a further aspect, the invention provides methods for prognosing breast cancer recurrence, comprising
(a) contacting a bodily fluid sample from a subject who has received breast cancer therapy with one or more reagents for detecting autoantibodies against one or more proteins selected from the group consisting of A1AT, ANGPTL4, LRP10, GFRA1, LGALS3, CST2, DKK1, CAPC, GRP78, and GRN; and
(b) determining an amount of autoantibodies against the one or more proteins in the bodily fluid sample;
wherein an increase in the amount of autoantibodies relative to a baseline level of autoantibodies in a control, such as a similar bodily fluid sample from the subject indicates a likelihood of breast cancer recurrence in the subject.

As disclosed in the examples that follow, a decrease in the amount of autoantibodies of the one or more recited markers over time compared to a baseline level (i.e.: before breast cancer therapy initiation) indicates a favorable treatment response, while no decrease, or an increase, in autoantibody levels indicates a non-favorable treatment response. The methods can be carried out at any suitable time after breast cancer therapy begins as determined by attending medical personnel in light of all factors. In various non-limiting embodiments, the methods may be carried out at least 1 week, 2 weeks, 3 weeks, 1 month, 2 months, 3 months, 4 months, 5 months, 6 months, 7 months, 8 months, 9 months, 10 months, 11 months, 12 months, 18 months, 24 months, etc. after the beginning of therapy. As will be understood by those of skill in the art, the methods can be carried out any number of times for a given subject as deemed appropriate by attending medical personnel. Thus, the methods can be carried out 1, 2, 3, 4, 5, 6, 7 8, 9, 10, or more times for a given subject, to monitor the course of therapy. In one non-limiting embodiment, the methods are carried out at 6 months and/or 12 months after initiation of breast cancer therapy. As will be understood by those of skill in the art, the methods can be carried out during the therapy, and can also be carried out after completion of the therapy, to monitor for possible breast cancer recurrence.

The subject may be any suitable subject receiving breast cancer therapy, including but not limited to a human subject. As used herein, "breast cancer therapy" includes one or more of surgery to remove the primary breast tumor, radiation therapy, chemotherapy, HERCEPTIN® therapy, and hormonal therapy. In one non-limiting embodiment, the subject has had surgery to remove the primary breast tumor and is receiving additional therapy selected from radiation therapy, chemotherapy, HERCEPTIN® therapy and/or hormonal therapy. Significant decreases in levels of response between baseline and 12 month time points against 9 TAAs (i.e. A1AT, ANGPTL4, CAPC, CST2, DKK1, GFRA1, GRN, LGALS3 and LRP10) were observed in three treatment groups. Radiation + chemotherapy, radiation + hormonal therapy, and radiation + hormonal therapy + chemotherapy had average geometric mean decreases for the 9 significant TAA of-11%, -13%, and -18%, respectively (Fig. 1). Autoantibodies against DKK1 significantly decreased in the radiation + chemotherapy group. The radiation + hormonal therapy group contained significant decreases in autoantibody response against A1AT, CST2, GRN and LRP10. In the Radiation + Hormonal therapy + chemotherapy group, A1AT and LRP10 had the greatest decrease in autoantibody response levels (-26% and -28%, respectively) compared to the other antigens and regimens. The triple therapy of radiation + hormonal + chemotherapy was more effective at reducing the autoantibody responses against the TAAs than any other combination of treatment. This group had significant decreases in autoantibody responses against all 9 TAAs.

In one embodiment, the one or more reagents comprise 2, 3, 4, or all 5 proteins, or antigenic fragments thereof, selected from the group consisting of ANGPTL4, GFRA1, LGALS3, DKK1 and GRN.

The antibody detection markers may be any suitable reagents that can be used to detect antibodies against the recited proteins, including but not limited to the recited protein, a secreted version of the protein (such as a native secreted form of the protein), or an extracellular domain of the protein. Secreted proteins are more easily delivered from tumor cells to lymph nodes, where interactions of immune cells take place resulting in abundant high-affinity antibodies. Membrane surface proteins are commonly released in a soluble form from tumor cells through metalloproteinase-dependent cleavage. The shed proteins are more easily transferred to the lymph nodes than intracellular protein. Thus, in one embodiment the antibody detection marker can be a secreted or membrane portion of the recited protein. Exemplary amino acid sequences of the secreted or membrane portion of the recited proteins are as disclosed herein. Thus, the reagents for use can be any suitable one or more reagents for detecting autoantibodies against one or more proteins selected from the group consisting of A1AT, ANGPTL4, LRP10, GFRA1, LGALS3, CST2, DKK1, CAPC, GRP78, and GRN, secreted portions thereof, or membrane portions thereof, including but not limited to the reagents of any embodiment of the compositions of the invention, or combinations thereof. In various non-limiting embodiments, the reagents comprise reagents for detecting human autoantibodies against at least three, four, five, six, seven, eight, or all nine proteins in the recited group. In another non-limiting embodiment, the reagents comprise the recited proteins or antigenic fragments thereof. Such proteins may be in a native form. In another embodiment, the reagents comprise at least two proteins, or antigenic fragments thereof, and wherein the at least two proteins, or antigenic fragments thereof, are expressed as a fusion with a detectable domain. The protein may be used in any suitable format; in one non-limiting embodiment, the protein(s) may be a surface-bound Fc fusion protein or secreted Fc fusion protein. In another embodiment, the reagents can be detectably labeled. In another embodiment, the reagents may be bound to a surface of a solid support.

In various embodiments, at least 1, 2, 3, 4, or 5 of the antibody detection markers are selected from the group consisting of A1AT, GFRA1, LGALS3, CAPC, and CST2. In various further embodiments, at least 1, 2, 3, 4, or 5 of the antibody detection markers are selected from the group consisting of ANGPTL4, GFRA1, LGALS3, DKK1 and GRN

As shown in the examples that follow, GRP78 was the only TAA to exhibit a significantly increase autoantibody response, which occurred in the hormonal therapy + chemotherapy group. Thus, in another aspect, the invention provides methods for monitoring breast cancer therapy, comprising
(a) contacting a bodily fluid sample from a subject who is undergoing or has undergone breast cancer therapy with one or more reagents for detecting autoantibodies against GRP78; and
(b) determining an amount of autoantibodies against GRP78 in the bodily fluid sample;
wherein an increase in the amount of autoantibodies against GRP78 relative to a control, such as a baseline level of autoantibodies in a similar bodily fluid sample from the subject indicates efficacy of the breast cancer therapy in the subject.

In another aspect, the invention provides methods for prognosing breast cancer recurrence, comprising
(a) contacting a bodily fluid sample from a subject who has received breast cancer therapy with one or more reagents for detecting autoantibodies against GRP78; and
(b) determining an amount of autoantibodies against GRP78 in the bodily fluid sample;
wherein a decrease in the amount of autoantibodies relative to a baseline level of autoantibodies against GRP78 in a control, such as a similar bodily fluid sample from the subject indicates a likelihood of breast cancer recurrence in the subject.

In one embodiment of each of these aspects, the subject is receiving or has received hormonal therapy and chemotherapy.

As will be understood by those of skill in the art, the methods may include the use of additional antibody detection markers and controls as is appropriate for an intended use of the composition. The contacting can be carried out under any suitable conditions for promoting binding between the autoantibodies in the bodily fluid sample and the reagent to forma binding complex that can be detected. Appropriate such conditions can be determined by those of skill in the art based on the intended assay, in light of the teachings herein. Similarly, any suitable additional steps can be used in the methods, such as one or more wash or other steps to remove unbound reagents.

Any suitable detection technique can be used, including but not limited to enzyme linked immunosorbent assays (ELISA), bead based assay platforms such as the LUMINEX® systems, 2-D array based assay platforms such as SEARCHLIGHT®, and the INANOVATE® 'Longitudinal Assay Screening' platform which may be capable of quantitating all the listed breast cancer biomarker from patient samples at their clinically relevant concentrations in a single test and dilution. In one embodiment, the compositions can be placed on a solid support, such as in a microarray, glass slide, membrane, microplate format or beads. The embodiment facilitates use of the compositions. Exemplary such assays are provided in the examples.

Similarly, any suitable bodily fluid can be used, including but not limited to a serum sample, plasma sample or blood sample from the subject. A "plasma sample" means blood plasma, the liquid component of blood, and is prepared, for example, by centrifugation of whole blood to remove blood cells. A serum sample is a plasma sample in which blood clotting factors have been removed.

In one embodiment, when no decrease is determined in the amount of autoantibodies relative to a baseline level of autoantibodies in a similar bodily fluid sample from the subject, the method further comprises altering the breast cancer therapy being administered to the subject. Since the lack of autoantibody decrease indicates a non-favorable therapeutic outcome for the subject, this embodiment permits modifying the therapy as deemed appropriate by attending medical personnel (i.e.: increased dosage, change in treatment, etc.) to achieve a more favorable therapeutic outcome.

In another embodiment, the methods of any embodiment or combination of embodiments are used to detect breast cancer recurrence. When no decrease (or an increase) is determined in the amount of autoantibodies relative to a baseline level of autoantibodies in a similar bodily fluid sample from the subject, the methods provide an indication of breast cancer recurrence. The fact that the autoantibody levels of patients decrease over the course of treatment supports the potential of using the detection of these autoantibody levels as a prognostic indication of recurrence. If the cancer recurs, the autoantibody levels increase and this increase would be detected by the assay.

### Example 1: Longitudinal Autoantibody Responses Against Tumor-Associated Antigens Decrease in Breast Cancer Patients According to Treatment Modality

Early diagnosis of breast cancer (BCa) is critical for increased disease survival, both at the time of initial disease as well as for recurrence (1, 2). The 5-year survival rate for women diagnosed with local BCa is 98.6%. Survival declines to 83.8% for regional stage and plummets to 23.3% for distant stage (3). Only 5% of U.S. women present with metastatic BCa when first diagnosed (4); however, incurable metastatic disease is responsible for most BCa-related deaths (5). Unfortunately, recurrence of BCa is most often found when patients report symptoms, such as shortness of breath, chronic cough, headache, weight loss or bone pain. Once a patient is diagnosed with metastatic BCa, the intent for treatment is no longer curative; instead, the goal is to control the disease for as long as possible (6, 7). Presently, no alternative screening methods are recommended for asymptomatic patients without clinical findings on physical examination (8). By the time the metastases are identified by physical symptoms, the patient's chances of disease-free survival are greatly diminished. Lack of early detection of recurrence for asymptomatic patients is a factor in the inability to cure metastatic BCa.

Early detection of the primary breast cancer (BCa) tumor allows physicians to treat a patient with the intent to cure. However, most BCa-related deaths are due to incurable metastatic disease and not the primary tumor. It is currently unknown what changes occur to patient autoantibody levels after primary diagnosis of BCa and treatment. A LUMINEX® multiplex bead assay was developed to allow simultaneous measurement of autoantibody responses against 32 conformation-carrying TAAs in a single patient sample. The antigens were selected from a membrane-associated polyribosomal cDNA library (MAPcL), which encodes membrane and secreted proteins highly expressed in BCa and should preferentially induce an antibody response in patients. The conformation of membrane and secreted proteins is particularly important because discontinuous epitopes will only be present for antibody recognition when the antigen is folded properly. Expression constructs were generated to encode the extracellular portion of the TAA fused to rabbit Fc (rFc). A eukaryotic expression system was developed to produce conformation-carrying antigens that are processed through the endoplasmic reticulum and Golgi to generate antigens with post-translational modifications.

LUMINEX® bead-based multiplex technology allows measurement of the interaction of patient autoantibodies with a panel of antigen biomarkers enabling quantitation across all biomarkers in a single test. The LUMINEX® xMAP® microsphere technology (Luminex, Austin, TX) is based on color-coded, 5.6-micron beads called microspheres. These beads are internally labeled with two different fluorescent dyes with different levels for each region, which are identified in the mixture by different red/infrared emission spectra. Simultaneously, the LUMINEX® instrument measures the amount of the autoantibody captured by the TAAs preloaded on the beads as the fluorescent level of the secondary antibody in a separate detector channel. The LUMINEX® beads have a much smaller surface area on which to immobilize the capture antibody as compared to the area of a microplate well; therefore, smaller patient sample volumes were required and non-specific binding to the plastic surfaces was reduced.

Three serial blood draws were collected from 200 BCa patients, before treatment, 6 and 12 months after surgery. These samples were assayed for autoantibody responses against 32 TAAs, and the levels present in the follow up samples were compared to autoantibody levels at the time of surgery. Patients were categorized according to treatment regimen, including surgery, chemotherapy, radiation, trastuzumab and hormonal therapies. Autoantibody responses against 9 TAAs (A1AT, ANGPTL4, CAPC, CST2, DKK1, GFRA1, GRN, LGALS3 and LRP10) were significantly reduced at 12 months after surgery with treatment regimens including radiation + chemotherapy, radiation + hormonal therapy, or radiation + hormonal + chemotherapy. Autoantibody levels were generally lower at 12 months than 6 months after surgery, consistent with the patient's tumor burden decreasing over the course of treatment. Consequently, this data indicates that autoantibody levels increase in response to metastasis, and detection of this change can be used as an early indicator of recurrence.

### MATERIALS AND METHODS

### Plasmid Construction and Protein Production

Briefly, the extracellular domain of transmembrane proteins or the full-length sequence of secreted and intracellular proteins was cloned into pSecTag2-rabbit Fc or pFUSE-rFc1. These plasmids included a secretion signal, as well as a C-terminal rabbit Fc (rFc) tag. The TAA-rFc plasmids were transfected into 293T cells with EFFECTENE™ (Qiagen, Valencia, CA), and the encoded proteins were secreted into the cell culture supernatant. Supernatants were harvested after transfection, and TAA-rFc content was measured with an anti-rFc sandwich ELISA.

### Antibody Coupling to Magnetic Beads

Goat anti-rabbit IgG Fc antibody (Jackson Immunoresearch, West Grove, PA) was coupled to LUMINEX® beads utilizing the xMAP® AbC Kit (Luminex, Austin, TX) according to the instruction of the manufacturer. In brief, beads were activated with EDC (1-Ethyl-3-[3-dimethylaminopropyl]carbodiimide hydrochloride) and Sulfo-NHS (*N-*hydroxysulfosuccinimide) for 20 minutes. After washing with phosphate buffered saline (PBS, pH 7.4), anti-rFc antibody was added to the beads at a concentration of 20 µg per 1 x 10⁶ beads and incubated for two hours shielded from light. The beads were washed again and stored at 4°C shielded from light until use. Coupling was performed on 32 LUMINEX® bead regions that can be mixed for use and distinguished in 32 different areas by a LUMINEX® 100/200 instrument (Millipore).

### Loading of Tumor-Associated Antigen-Rabbit Fc Fusion Proteins to Beads

Beads coupled with the anti-rFc antibody were coated with each TAA-rFc fusion protein by incubation with the cell culture supernatant containing secreted TAA-rFc fusion protein. Each of the 32 TAA-rFc fusions was bound to one LUMINEX® bead region. Beads were incubated with the fusion protein at 40 µg/10⁶ beads overnight at 4°C. Beads were stored in PBS-TBN buffer (PBS with 0.1% bovine serum albumin, 0.02% Tween 20 and 0.05% sodium azide) at 4°C in the dark until use.

### Patients

The inclusion criteria for cases were women over 30 years of age that were newly diagnosed with BCa (any type) at Sanford Health, Sioux Falls, SD. Patients provided one 10 ml EDTA tube of blood prior to mastectomy, lumpectomy, radiation therapy, chemotherapy or other treatment. Potential subjects were excluded if they had a previous history of cancer of any kind. Blood samples from 200 BCa patients were collected from 10/08/09 to 4/17/12. Patients enrolled in the study were followed for one year after surgery, and blood draws were obtained at follow-up oncology visits at 6 and 12 months after surgery. Follow-up draws were collected from 4/15/10 to 8/27/13. All patients provided written informed consent, and the Sanford Health IRB approved the study protocol.

### Plasma Collection and storage

The 10 ml EDTA tube was centrifuged at 2000 x g for 10 minutes within 12 hrs of drawing. Plasma was collected as the supernatant, placed in aliquots and stored at -80°C until the assay for the autoantibodies.

### Multiplex Autoantibody Bead Assay

Autoantibodies against the 32 TAAs in the plasma samples were measured simultaneously in a single well utilizing a multiplex bead assay. The xMAP® LUMINEX® magnetic beads from 32 distinct regions, each coated with a different TAA-rFc fusion, were combined and distributed to the wells of a 96-well round bottom plate. Plasma samples from BCa patients were diluted 1:10 in FACS buffer (PBS with 5% fetal bovine serum and 0.1% sodium azide), and 200 µl of diluted plasma was applied to the beads in a single well. Samples were incubated with beads for 2 hours on ice. Beads were then pelleted magnetically and washed twice, with a final aspiration leaving only the beads. As the secondary antibody, R-Phycoerythrin (PE)-labeled goat anti-human IgG (Jackson Immunoresearch, West Grove, PA) was diluted 1:200 in FACS buffer and 200 µl was added to the beads of each well. After an incubation of one hour on ice, beads were washed twice. The beads in each well were resuspended in 200 µl of FACS buffer and analyzed on a LUMINEX® 100/200 instrument, with a minimum of 100 events analyzed for each bead region. Each plate included a secondary only negative control, as well as a PE goat anti-rabbit IgG (Jackson Immunoresearch, West Grove, PA) reacting with bead-loaded TAA-rFc fusions as a positive control. All washing and aspiration steps were performed with a Biotek ELx405 microplate washer with magnetic capabilities.

### Statistical Methods

Each 96-well plate had a negative control *background* and a positive control *standard* for all autoantibodies. In addition, each patient had her baseline, 6 month and 12 month samples analyzed in the same 96-well plate. For each autoantibody, the patient's median fluorescent intensity (MFI) value had its plate MFI background level subtracted and was then shifted by the minimum constant to make all values at least one. The value was then normalized by the ratio of the MFI for the standard over the mean standard MFI across all 8 plates. Lastly the values were log transformed to stabilize the variance and produce a more symmetric distribution, i.e. autoantibody response = LN[(autoantibody - background + constant)*(standard - background) / mean(standard - background)]. To measure precision, the inter-assay CV for each TAA was calculated across the plates for the positive and negative controls.

A repeated measure ANOVA was used to model the geometric mean changes from baseline for each autoantibody over time (i.e. 6 and 12 months) as an exploratory analysis, with a compound symmetry correlation structure. The models included all interactions among indicator variables for radiation, hormonal and chemotherapy with time. This approach included 183 out of 200 BCa patients in the primary analysis; the 17 patients given trastuzumab (HERCEPTIN®) as part of their treatment consisted of 4 groups and were analyzed secondarily by examining their mean response profile. Point estimates and 95% confidence intervals (CI) were calculated for all 8 treatment combinations, which did not include HERCEPTIN®, at 6 and 12 months in order to rank autoantibodies by the number of positive findings. Since each TAA had 16 (8 treatments at 6 and 12 months) point estimates tested, one false positive was expected for each TAA; therefore, the observed geometric mean changes were presented for autoantibody levels with 3 or more positive findings. A p-value < 0.05 was used to ascribe statistical significance, and SAS (Cary, NC) version 9.3 was used for all analyses.

### RESULTS

Serial plasma samples were collected from 200 newly diagnosed BCa patients (13). Characteristics of the 200 BCa patients enrolled in this study have been previously described by our laboratory, including demographic information, tumor size, tumor marker status, *in situ* versus invasive components and lymph node involvement (13). Blood draws were acquired before treatment, 6 months and 12 months after surgical resection of the primary tumor. To determine the patients' autoantibody responses against cancer antigens over the course of treatment, 32 TAA-rFc fusion proteins consisting of 20 previously analyzed TAAs (13) and 12 newly selected cancer antigens (**Table 1**) were generated. The eukaryotic expression system developed previously (13) was used to generate all of the TAA-rFc conformation-carrying antigens for the multiplex immunoassay. Thirty-two sets of LUMINEX® beads consisting of unique red/infrared emission spectra were coated with anti-rabbit IgG. The 32 TAA-rFc fusion proteins were attached to the coated LUMINEX® beads followed by incubation with plasma samples acquired from patients before treatment, 6 months and 12 months post-surgery. The average inter-assay CV for the 32 autoantibody responses measured at baseline (before the start of treatment) with the LUMINEX® multiplex bead platform were 11.1% and 11.4% for the low and high controls, respectively (**Table 2**). In comparison, the average inter-assay CV for the blocking buffer control of the ELISA platform autoantibody assay was 12.4% (13). The LUMINEX® multiplex system greatly increased assay throughput and slightly improved the reproducibility.

**Table 1. Additional candidates for generation of rFc fusion proteins**

| **Gene** | **Accession #** | **Signal Sequence Amino Acids** | **Encoded Amino Acid Fragment** |
|---|---|---|---|
| A1AT | NM_000295.4 | 1-24 | 25-418 |
| AMACR | NM_014324.5 | None | 1-382 |
| BIRC5 | NM_001168.2 | None | 1-142 |
| CALD1 | NM_033139.3 | None | 1-558 |
| CAPC | NM_001013653.2 | 1-26 | 27-264 |
| CCNB1 | NM_031966.3 | None | 1-433 |
| CCND1 | NM_053056.2 | None | 1-295 |
| GRP78 | NM_005347.4 | None | 1-654 |
| LGALS3 | NM_002306.3 | None | 1-250 |
| MYC | NM_002467.4 | None | 1-439 |
| NY-ESO-1 | NM_001327.2 | None | 1-180 |
| XAGE1 | NM_001097594.2 | None | 1-81 |

**Table 2. Inter-assay coefficient of variation for negative and positive controls for 32 TAA on the bead platform**

| **Autoantibody** | **Negative Control (Background)** | **Positive Control (Standard)** |
|---|---|---|
| | **Original 20** | |
| ANGPTL4 | 9.2 | 12.3 |
| CD147 | 5.7 | 10.7 |
| CD320 | 21.8 | 12.9 |
| CDH3 | 7.5 | 12.3 |
| CST2 | 13.4 | 12.2 |
| DKK1 | 6.6 | 9.7 |
| EPHA2 | 5.2 | 12.6 |
| GFRA1 | 9.6 | 11.5 |
| GRN | 11.7 | 15.4 |
| IGFBP2 | 14.7 | 9.7 |
| LAMC2 | 7.7 | 11.1 |
| LRP10 | 5.5 | 10.3 |
| LRRC15 | 5.7 | 16.1 |
| MUC1 | 8.3 | 10.1 |
| SPINT2 | 12.9 | 9.2 |
| SPON2 | 14.3 | 10.5 |
| SSR2 | 18.2 | 11.2 |
| SUSD2 | 7.0 | 12.3 |
| LGALS1 | 14.7 | 11.7 |
| ERBB2 | 9.3 | 12.2 |

| | **Additional 12** | |
|---|---|---|
| A1AT | 12.2 | 12.8 |
| AMACR | 11.3 | 11.2 |
| BIRC5 | 12.8 | 11.3 |
| CALD1 | 11.8 | 9.9 |
| CAPC | 10.2 | 10.4 |
| CCNB1 | 12.8 | 9.2 |
| CCND1 | 10.9 | 10.3 |
| GRP78 | 11.9 | 11.7 |
| LGALS3 | 13.1 | 13.9 |
| MYC | 12.5 | 9.5 |
| NY-ESO-1 | 15.9 | 11.2 |
| XAGE1 | 11.3 | 10.9 |
| **Average** | 11.1 | 11.4 |

Each subject was categorized based on the treatments received during the 12 months following initial diagnosis. All enrolled BCa patients in our study underwent surgery to remove the primary tumor, including a breast lumpectomy or mastectomy. In addition to surgery, treatment included combinations of hormonal therapies, HERCEPTIN®, radiation and cytotoxic chemotherapy (**Table 3**). Seventy-two patients received cytotoxic chemotherapy consisting of one of the following regimens: adriamycin/cytoxan + taxol = 33 patients; cytoxan + taxol = 22 patients; adriamycin/cytoxan = 5 patients; carboplatin + taxol = 6 patients; adriamycin + cytoxan/taxol = 3 patients; adriamycin/cytoxan + carboplatin/Gemzar = 1 patient; carboplatin + taxotere + novantrone = 1 patient; taxol alone = 1 patient. Patients were not analyzed according to the subclass of cytotoxic chemotherapy drugs used for treatment. If a patient had any one of the chemotherapy regiments described above, they were considered part of the chemotherapy treatment group. Grouping the study participants based on treatment regimen resulted in 12 groups, ranging from no treatment after surgery to all treatment modalities administered (**Table 3**). Eight of the twelve groups received a combination of at least two therapies in addition to surgery. Three groups received a single therapy in addition to surgery, and one group received surgery alone.

**Table 3. Number of Patient Blood Draws Per Treatment Modality and Visit**

| **Treatment Group*** | **Baseline** | **6 Month** | **12 Month** | **Subtotal** |
|---|---|---|---|---|
| Radiation + Hormonal | 59 (29.5%) | 52 | 52 | 163 |
| Hormonal | 31 (15.5%) | 25 | 26 | 82 |
| Radiation + Hormonal + Chemotherapy | 25 (12.5%) | 23 | 22 | 70 |
| Surgery Only | 24 (12.0%) | 15 | 10 | 49 |
| Hormonal + Chemotherapy | 15 (7.5%) | 12 | 13 | 40 |
| Radiation | 13 (6.5%) | 8 | 8 | 29 |
| Radiation + Chemotherapy | 11 (5.5%) | 11 | 11 | 33 |
| Radiation + Hormonal + Chemotherapy + HERCEPTIN® | 8 (4.0%) | 8 | 8 | 24 |
| Chemotherapy | 5 (2.5%) | 4 | 5 | 14 |
| Hormonal + Chemotherapy + HERCEPTIN® | 5 (2.5%) | 5 | 5 | 15 |
| Radiation + Chemotherapy + HERCEPTIN® | 3 (1.5%) | 2 | 2 | 7 |
| Radiation + Hormonal + HERCEPTIN® | 1 (0.5%) | 1 | 1 | 3 |
| Total number of samples | 200 (100%) | 166 | 163 | 529 |

| | | | | |
|---|---|---|---|---|
| *All patients received surgery to remove the primary tumor | | | | |

The primary exploratory analysis included 183 out of 200 patients and encompassed the 8 patient treatment groups not receiving HERCEPTIN® therapy (**Table 3**). A repeated measure ANOVA was used to model the geometric mean changes from baseline for each autoantibody at 6 and 12 months, and the model included all interactions among indicator variables for radiation, hormonal therapy and chemotherapy with time. If the ANOVA model predicted at least 3 significant changes for an antigen among the 16 estimates (8 groups * 2 time points consisting of 6 and 12 month blood draws), it was chosen for further analysis. Using this variable selection criterion, the model identified 11 antigens that were likely to have significantly modulated autoantibody signals in response to treatment in the 12 months following surgery. The TAAs chosen for further study included: A1AT, ANGPTL4, CAPC, CST2, DKK1, GFRA1, GRN, GRP78, LGALS3, LRP10 and NY-ESO-1.

The actual geometric mean changes from baseline were calculated for the 11 TAAs at 6 and 12 months (**Fig. 1** and **3**, respectively). No significant changes in the autoantibody response against the 11 antigens were observed for patients who received surgery alone or were treated with surgery and a single monotherapy: hormonal therapy, chemotherapy or radiation. At the 12-month time point, GRP78 was the only TAA to exhibit a significant increase autoantibody response of 44%, which occurred in the hormonal therapy + chemotherapy group (Fig. 1). While NY-ESO-1 was predicted to have significant changes in levels of response in the repeated measures ANOVA model, the observed changes were not significant (**Fig. 1**). However, significant decreases in levels of response between baseline and 12 month time points against 9 of the 11 TAAs (i.e. A1AT, ANGPTL4, CAPC, CST2, DKK1, GFRA1, GRN, LGALS3 and LRP10) were observed in three treatment groups. Radiation + chemotherapy, radiation + hormonal therapy, and radiation + hormonal therapy + chemotherapy had average geometric mean decreases for the 9 significant TAA of-11%, - 13%, and -18%, respectively (Fig. 1). In the Radiation + Hormonal therapy + chemotherapy group, A1AT and LRP10 had the greatest decrease in autoantibody response levels (-26% and -28%, respectively) compared to the other antigens and regimens. The triple therapy of radiation + hormonal + chemotherapy was more effective at reducing the autoantibody responses against the TAAs than any other combination pair of treatment. In addition, the decrease in response of the geometric mean was -15% at 6 months after surgery (**Fig. 3**) compared to a greater decrease of -18% at the 12 month time point. It is apparent that autoantibody levels diminish during the course of treatment, and they continue to decrease with time as the patient is further removed from initial diagnosis.

Four of the 12 treatment groups (totaling 17 patients) were treated with HERCEPTIN® therapy as follows: radiation + hormonal + chemotherapy + HERCEPTIN® = 8 patients; hormonal + chemotherapy + HERCEPTIN® = 5 patients; radiation + chemotherapy + HERCEPTIN® = 3 patients; radiation + hormonal + HERCEPTIN® = 1 patient (Table 2). HERCEPTIN® antibody therapy is generally administered every three weeks for one year (27). We confirmed that the HERCEPTIN® antibody circulating in the patient's blood was generating a signal against the ERBB2 antigen using the LUMINEX® bead based assay. The geometric mean levels in the ERBB2 antibody response increased from 48 median fluorescence intensity (MFI) at baseline to 1206 MFI at 6 months, and then decreased to 600 MFI at 12 months (**Fig. 2**). The changes compared to baseline were significant at 6 and 12-months (both p<0.0001), but there was no significant difference between 6 and 12-month levels (p=0.09). Most patients had a high response against ERBB2 at the 6-month time point, which is consistent with the fact that patients were receiving HERCEPTIN® therapy spanning this time period. Patient BC-166 (Fig. 2, light green circle) received her treatments at an off-site medical center, and therefore, the 6 and 12-month blood draws were not acquired. Six patients (**Fig. 2**, dashed lines) discontinued HERCEPTIN® therapy prior to their 12-month visit. Subject BC-021 had very low antibody response against ERBB2, which is consistent with her only receiving two treatments before discontinuing therapy. For the other five patients that had discontinued HERCEPTIN® therapy, their anti-ERBB2 antibody responses for the 12-month blood draws were inversely related to the number of months from the patients' last HERCEPTIN® infusions. Patient BC-082 discontinued HERCEPTIN® use after her 6-month visit, and by her 12-month visit the MFI value had returned to baseline. Patients BC-149, BC-013 and BC-84 were 4 months removed from treatment and had a level of 110 MFI, 244 MFI and 994 MFI, respectively. Patient BC-016 had her final blood draw at 3 months after her last HERCEPTIN® infusion, and her response level was 418 MFI. BC-013 had a strong endogenous autoantibody response to ERBB2 prior to treatment with HERCEPTIN®, yielding a high signal at the baseline time point (**Fig. 2**). The remaining 10 patients (BC-018, BC-019, BC-033, BC-038, BC-051, BC-054, BC-130, BC-158, BC-188 and BC-189) were continuing to receive HERCEPTIN® during the longitudinal blood draws, and the anti-ERBB2 antibody response against the ERBB2 antigen plateaued between the 6 and 12-month visits (**Fig. 2**).

### DISCUSSION

This study provides important data on longitudinal change of autoantibody responses occurring in BCa patients after surgery. We were able to assess many TAAs simultaneously with our unique multiplex bead assay. In addition, rich data on pathology and treatment information allows the findings of this study to be associated with the treatment regimens administered to each patient. Our results indicate that the combination of the selected 9 antigens shows promise in developing an autoantibody assay toward early detection of metastatic disease.

The data collected from the analysis of longitudinal blood draws in this study indicates that autoantibody response to TAAs decreases as a function of time after the primary tumor is surgically resected and therapy is initiated (**Fig. 1**). The absence of a large tumor mass likely explains the reduced production of autoantibodies to TAAs. With the majority of the cancer cells removed, the immune system is exposed to fewer cancer antigens. Without continued exposure to the cancer antigens, the immune system reduces the levels of autoantibody production. Surprisingly, we also found that treatments administered after the removal of the primary tumor had a profound effect on the autoantibody profile of these patients. Significant changes in antibody responses can be detected at the 6-month time point (**Table 2**), with a more extensive reduction detectable 12 months after surgery (**Table 3**). The changes seen in these samples are attributed to the therapies administered to that patient, but it is acknowledged that these therapies are a function of the characteristics of each subtype of BCa.

To that end, the treatment modality groups that had the greatest decrease in autoantibody response levels were radiation + hormonal therapy; radiation + chemotherapy; and radiation + hormonal therapy + chemotherapy (**Fig. 1**). The common denominator of the three most affected groups for significant changes in autoantibody response levels is radiation treatment. However, radiation treatment alone is not enough to significantly decrease the response levels of the autoantibodies. Patients treated with a combination of all therapies (surgery, hormonal therapy, chemotherapy and radiation), had autoantibody response levels significantly decrease for 8 separate antigens (**Fig. 1**). Finally, the hormonal therapy + chemotherapy regimen contained a significant change in an autoantibody response against the GRP78 antigen. This group of patients did not receive radiation therapy, and the autoantibody response against GRP78 was unique because instead of decreasing, the response dramatically increased 12 months after surgery (**Fig. 1**).

The initial analysis of the data revealed that a group of patients had a high response against the ERBB2 antigen (**Fig. 2**). Upon further evaluation, we determined that the tumors removed from those patients were HER-2 amplified, and the patients were candidates for HERCEPTIN® therapy. HERCEPTIN® antibody recognizes and binds to a native pocket-like binding region of ERBB2 (33, 34). By using our conformation-carrying method of antigen generation, an ERBB2-rFc fusion protein was produced which mimicked the conformation of the native ERBB2 protein (13). Our assay was measuring the response of the HERCEPTIN® therapeutic antibody present in the peripheral circulation of the patient against the ERBB2 antigen attached to the beads (**Fig. 2**). Since HERCEPTIN® therapy is generally administered every three weeks by infusion for one year (27), this response served as an '*in vivo* human spiked control' for our multiplex bead assay. HERCEPTIN® antibody present in the circulation of patients produced a high level of response against the ERBB2-rFc fusion protein corresponding to the timing of HERCEPTIN® treatments of patients. Previously, a phase II study determined that the half-life of trastuzumab was 18 to 27 days when administered every 3 weeks (35). This length of time is consistent with the measured levels of the anti-ERBB2 antibody response over the course and end of trastuzumab treatment for the patients with HER-2 amplified tumors. For patients that had their final blood draw 3 and 4 months after finishing trastuzumab treatment, their antibody response against ERBB2 decreased dramatically. One patient had her blood drawn 6 months following her last trastuzumab infusion, and her response had returned to pretreatment levels (**Fig. 2**).

Of the 9 antigens reported in Figure 2 whose autoantibody responses were significantly lowered over the course of treatment (A1AT, ANGPTL4, CAPC, CST2, DKK1, GFRA1, GRN, LGALS3 and LRP10), 4 were also present in the group of 7 antigens previously found to be predictive of the presence of BCa: ANGPTL4, DKK1, GFRA1 and GRN (13).

### REFERENCES

1. Aragon R, Morgan J, Wong JH, Lum S. Potential impact of USPSTF recommendations on early diagnosis of breast cancer. Annals of surgical oncology. 2011;18:3137-42.
2. Kontos M, Roy P, Rizos D, Petrou A, Hamed H. Contralateral relapse after surgery for breast cancer: evaluation of follow-up paradigms. International journal of clinical practice. 2013;67:1113-7.
3. Siegel R, DeSantis C, Virgo K, Stein K, Mariotto A, Smith T, et al. Cancer treatment and survivorship statistics, 2012. CA Cancer J Clin. 2012;62:220-41.
4. American Cancer Society. Breast Cancer Facts & Figures 2013-2014. [cited 2014 03/27/14]; Available from: http://www.cancer.org
5. Lu J, Steeg PS, Price JE, Krishnamurthy S, Mani SA, Reuben J, et al. Breast cancer metastasis: challenges and opportunities. Cancer Res. 2009;69:4951-3.
6. Kimbung S, Loman N, Hedenfalk I. Clinical and molecular complexity of breast cancer metastases. Seminars in cancer biology. 2015.
7. Cardoso F, Costa A, Norton L, Cameron D, Cufer T, Fallowfield L, et al. 1st International consensus guidelines for advanced breast cancer (ABC 1). Breast. 2012;21:242-52.
8. Schneble EJ, Graham LJ, Shupe MP, Flynt FL, Banks KP, Kirkpatrick AD, et al. Current approaches and challenges in early detection of breast cancer recurrence. Journal of Cancer. 2014;5:281-90.
9. Chapman C, Murray A, Chakrabarti J, Thorpe A, Woolston C, Sahin U, et al. Autoantibodies in breast cancer: their use as an aid to early diagnosis. Annals of oncology : official journal of the European Society for Medical Oncology / ESMO. 2007;18:868-73.
10. Lacombe J, Mange A, Solassol J. Use of autoantibodies to detect the onset of breast cancer. Journal of immunology research. 2014;2014:574981.
11. Henry NL, Hayes DF, Ramsey SD, Hortobagyi GN, Barlow WE, Gralow JR. Promoting quality and evidence-based care in early-stage breast cancer follow-up. Journal of the National Cancer Institute. 2014;106:dju034.
12. Egland KA, Vincent JJ, Strausberg R, Lee B, Pastan I. Discovery of the breast cancer gene BASE using a molecular approach to enrich for genes encoding membrane and secreted proteins. Proc Natl Acad Sci USA. 2003;100:1099-104.
13. Evans RL, Pottala JV, Egland KA. Classifying Patients for Breast Cancer by Detection of Autoantibodies against a Panel of Conformation-Carrying Antigens. Cancer Prev Res 2014.
14. Carson RT, Vignali DA. Simultaneous quantitation of 15 cytokines using a multiplexed flow cytometric assay. J Immunol Methods. 1999;227:41-52.
15. Egland KA, Liu XF, Squires S, Nagata S, Man YG, Bera TK, et al. High expression of a cytokeratin-associated protein in many cancers. Proc Natl Acad Sci USA. 2006;103:5929-34.
16. Fossa A, Alsoe L, Crameri R, Funderud S, Gaudernack G, Smeland EB. Serological cloning of cancer/testis antigens expressed in prostate cancer using cDNA phage surface display. Cancer immunology, immunotherapy : CII. 2004;53:431-8.
17. Gonzalez-Gronow M, Cuchacovich M, Llanos C, Urzua C, Gawdi G, Pizzo SV. Prostate cancer cell proliferation in vitro is modulated by antibodies against glucose-regulated protein 78 isolated from patient serum. Cancer Res. 2006;66:11424-31.
18. Liu W, De La Torre IG, Gutierrez-Rivera MC, Wang B, Liu Y, Dai L, et al. Detection of autoantibodies to multiple tumor-associated antigens (TAAs) in the immunodiagnosis of breast cancer. Tumour Biol. 2015;36:1307-12.
19. Lopez-Arias E, Aguilar-Lemarroy A, Felipe Jave-Suarez L, Morgan-Villela G, Mariscal-Ramirez I, Martinez-Velazquez M, et al. Alpha 1-antitrypsin: a novel tumor-associated antigen identified in patients with early-stage breast cancer. Electrophoresis. 2012;33:2130-7.
20. O'Rourke DJ, DiJohnson DA, Caiazzo RJ, Jr., Nelson JC, Ure D, O'Leary MP, et al. Autoantibody signatures as biomarkers to distinguish prostate cancer from benign prostatic hyperplasia in patients with increased serum prostate specific antigen. Clinica chimica acta; international journal of clinical chemistry. 2012;413:561-7.
21. Xie C, Kim HJ, Haw JG, Kalbasi A, Gardner BK, Li G, et al. A novel multiplex assay combining autoantibodies plus PSA has potential implications for classification of prostate cancer from non-malignant cases. Journal of translational medicine. 2011;9:43.
22. Xu Y, Jin Y, Liu L, Zhang X, Chen Y, Wei J. Study of circulating IgG antibodies to peptide antigens derived from BIRC5 and MYC in cervical cancer. FEBS open bio. 2015;5:198-201.
23. Yang Z, Chevolot Y, Gehin T, Solassol J, Mange A, Souteyrand E, et al. Improvement of protein immobilization for the elaboration of tumor-associated antigen microarrays: application to the sensitive and specific detection of tumor markers from breast cancer sera. Biosensors & bioelectronics. 2013;40:385-92.
24. Ye H, Sun C, Ren P, Dai L, Peng B, Wang K, et al. Mini-array of multiple tumor-associated antigens (TAAs) in the immunodiagnosis of breast cancer. Oncology letters. 2013;5:663-8.
25. Zhou SL, Yue WB, Fan ZM, Du F, Liu BC, Li B, et al. Autoantibody detection to tumor-associated antigens of P53, IMP1, P16, cyclin B1, P62, C-myc, Survivn, and Koc for the screening of high-risk subjects and early detection of esophageal squamous cell carcinoma. Diseases of the esophagus : official journal of the International Society for Diseases of the Esophagus / ISDE. 2014;27:790-7.
26. Zuo X, Chen L, Liu L, Zhang Z, Zhang X, Yu Q, et al. Identification of a panel of complex autoantigens (LGALS3, PHB2, MUC1, and GK2) in combination with CA15-3 for the diagnosis of early-stage breast cancer. Tumour Biol. 2015.
27. HERCEPTIN® Prescribing Information. Genentech, Inc. 2015.
28. Heo CK, Bahk YY, Cho EW. Tumor-associated autoantibodies as diagnostic and prognostic biomarkers. BMB reports. 2012;45:677-85.
29. Liu W, Peng B, Lu Y, Xu W, Qian W, Zhang JY. Autoantibodies to tumor-associated antigens as biomarkers in cancer immunodiagnosis. Autoimmun Rev. 2011;10:331-5.
30. Zhu Q, Liu M, Dai L, Ying X, Ye H, Zhou Y, et al. Using immunoproteomics to identify tumor-associated antigens (TAAs) as biomarkers in cancer immunodiagnosis. Autoimmun Rev. 2013;12:1123-8.
31. Fernandez-Madrid F, Maroun MC. Autoantibodies in breast cancer. Advances in clinical chemistry. 2014;64:221-40.
32. Lu H, Goodell V, Disis ML. Humoral Immunity Directed against Tumor-Associated Antigens As Potential Biomarkers for the Early Diagnosis of Cancer. J Proteome Res. 2008;7:1388-94.
33. Cho HS, Mason K, Ramyar KX, Stanley AM, Gabelli SB, Denney DW, Jr., et al. Structure of the extracellular region of HER2 alone and in complex with the Herceptin Fab. Nature. 2003;421:756-60.
34. Garrett JT, Rawale S, Allen SD, Phillips G, Forni G, Morris JC, et al. Novel engineered trastuzumab conformational epitopes demonstrate in vitro and in vivo antitumor properties against HER-2/neu. J Immunol. 2007;178:7120-31.
35. Leyland-Jones B, Gelmon K, Ayoub JP, Arnold A, Verma S, Dias R, et al. Pharmacokinetics, safety, and efficacy of trastuzumab administered every three weeks in combination with paclitaxel. J Clin Oncol. 2003;21:3965-71.

### The following items disclose specific embodiments of the invention

1. A composition comprising at least 2 antibody detection markers, wherein the antibody detection markers comprise reagents for detecting human autoantibodies against at least two proteins selected from the group consisting of A1AT (Alpha-1 antitrypsin) (SEQ ID NO:7), ANGPTL4 (Angiopoietin-like 4) (SEQ ID NO:1), LRP10 (LDL Receptor Related Protein 10) (SEQ ID NO:5), GFRA1 (GDNF Family Receptor Alpha 1) (SEQ ID NO:3), LGALS3 (Galectin-3) (SEQ ID NO:8), CST2 (Cystatin SA) (SEQ ID NO:6), DKK1 (Dickkopf WNT Signaling Pathway Inhibitor 1) (SEQ ID NO:2), CAPC (Cytokeratin-Associated Protein In Cancer) (SEQ ID NO:9), GRP78 (78 kDa glucose-regulated protein) (SEQ ID NO:12) and GRN (Granulin) (SEQ ID NO:4), wherein at least one of the proteins is selected from the group consisting of A1AT, LGALS3, and CAPC.
2. The composition of item 1, wherein the composition includes reagents for detecting human autoantibodies against at least 3 proteins selected from the group consisting of A1AT, ANGPTL4, LRP10, GFRA1, LGALS3, CST2, DKK1, CAPC, GRP78, and GRN.
3. The composition of item 1 or 2, wherein the composition includes reagents for detecting human autoantibodies against at least 5, 6, 7, 8, or all 9 proteins in the recited group.
4. The composition of any one of items 1-3, wherein the composition consists of between 2 and 1000 antibody detection markers.
5. The composition of any one of items 1-3, wherein the composition consists of between 4 and 500 antibody detection markers.
6. The composition of any one of items 1-5, wherein the composition includes reagents for detecting human autoantibodies against at least 2 of ANGPTL4, GFRA1, LGALS3, DKK1 and GRN.
7. The composition of any one of items 1-5, wherein the composition includes reagents for detecting human autoantibodies against at least 3, 4, or all 5 of ANGPTL4, GFRA1, LGALS3, DKK1 and GRN.
8. The composition of any one of items 1-7, wherein the reagents for detecting human autoantibodies comprise the at least two proteins, or antigenic fragments thereof.
9. The composition of item 8, wherein the at least two proteins or antigenic fragments thereof comprise native extracellular domains and/or native secreted proteins or antigenic fragments thereof.
10. The composition of any one of items 1-9, wherein the reagents are detectably labeled.
11. The composition of any one of items 8-10, wherein the at least two proteins, or antigenic fragments thereof, are expressed as a fusion with a detectable domain, including but not limited to an Fc domain.
12. The composition of any one of items 1-11, wherein the reagents are immobilized on a surface of a solid support.
13. A method for monitoring breast cancer therapy, comprising
   (a) contacting a bodily fluid sample from a subject who is undergoing or has undergone breast cancer therapy with one or more reagents for detecting autoantibodies against one or more proteins selected from the group consisting of A1AT, ANGPTL4, LRP10, GFRA1, LGALS3, CST2, DKK1, CAPC, GRP78, and GRN; and
   (b) determining an amount of autoantibodies against the one or more proteins in the bodily fluid sample;
   wherein a decrease in the amount of autoantibodies relative to a control, such as a baseline level of autoantibodies in a similar bodily fluid sample from the subject indicates efficacy of the breast cancer therapy in the subject.
14. A method for prognosing breast cancer recurrence, comprising
   (a) contacting a bodily fluid sample from a subject who has received breast cancer therapy with one or more reagents for detecting autoantibodies against one or more proteins selected from the group consisting of A1AT, ANGPTL4, LRP10, GFRA1, LGALS3, CST2, DKK1, CAPC, GRP78, and GRN; and
   (b) determining an amount of autoantibodies against the one or more proteins in the bodily fluid sample;
   wherein an increase in the amount of autoantibodies relative to a baseline level of autoantibodies in a control, such as a similar bodily fluid sample from the subject indicates a likelihood of breast cancer recurrence in the subject.
15. The method of any one of items 13-14, wherein the reagents comprise reagents for detecting autoantibodies 3 or more of the recited proteins.
16. The method of any one of items 12-13, wherein the reagents comprise reagents for detecting autoantibodies against 5, 6, 7, 8, or all 9 of the recited proteins.
17. The method of any one of items 13-16, wherein the one or more reagents comprise the composition of any one of claims 1-12.
18. The method of any one of items 13-16, wherein the one or more reagents comprise at least 2 proteins, or antigenic fragments thereof, selected from the group consisting of ANGPTL4, GFRA1, LGALS3, DKK1 and GRN.
19. The method of any one of items 13-16, wherein the one or more reagents comprise at least 3, 4, or all 5 proteins, or antigenic fragments thereof, selected from the group consisting of ANGPTL4, GFRA1, LGALS3, DKK1 and GRN.
20. A method for monitoring breast cancer therapy, comprising
   (a) contacting a bodily fluid sample from a subject who is undergoing or has undergone breast cancer therapy with one or more reagents for detecting autoantibodies against GRP78 (SEQ ID NO:12); and
   (b) determining an amount of autoantibodies against GRP78 in the bodily fluid sample;
   wherein an increase in the amount of autoantibodies against GRP78 relative to a control, such as a baseline level of autoantibodies in a similar bodily fluid sample from the subject indicates efficacy of the breast cancer therapy in the subject.
21. A method for prognosing breast cancer recurrence, comprising
   (a) contacting a bodily fluid sample from a subject who has received breast cancer therapy with one or more reagents for detecting autoantibodies against GRP78 (SEQ ID NO:12); and
   (b) determining an amount of autoantibodies against GRP78 in the bodily fluid sample;
   wherein a decrease in the amount of autoantibodies relative to a baseline level of autoantibodies against GRP78 in a control, such as a similar bodily fluid sample from the subject indicates a likelihood of breast cancer recurrence in the subject.
22. The method of any one of items 13-21, wherein the contacting comprises use of ELISA.
23. The method of any one of items 13-22, wherein the bodily fluid sample comprises a blood sample, a plasma sample, or a serum sample from the subject.
24. The method of any one of items 13-23, wherein the subject has had surgery to remove the primary tumor.
25. The method of any one of items 13-24, wherein the subject is receiving or has received radiation therapy and chemotherapy.
26. The method of any one of items 13-25, wherein the contacting comprises use of Longitudinal Assay Screening, wherein all target biomarkers may be detected and quantitated within a single test and dilution.
27. The method of any one of items 20-24 and 26, wherein the subject is receiving or has received hormonal therapy and chemotherapy.
28. The method of any one of items 13-27, wherein no decrease is determined in the amount of autoantibodies relative to a baseline level of autoantibodies in a similar bodily fluid sample from the subject, and wherein the method further comprises altering the breast cancer therapy being administered to the subject.

## Claims

1. A composition comprising at least 2 antibody detection markers, wherein the antibody detection markers comprise at least two proteins, or antigenic fragments thereof, comprising:
(a) DKK1 (Dickkopf WNT Signaling Pathway Inhibitor 1) (SEQ ID NO:2); and
(b) one or more proteins, or antigenic fragments thereof, selected from the group consisting of A1AT (Alpha-1 antitrypsin) (SEQ ID NO:7), ANGPTL4 (Angiopoietin-like 4) (SEQ ID NO:1), LRP10 (LDL Receptor Related Protein 10) (SEQ ID NO:5), GFRA1 (GDNF Family Receptor Alpha 1) (SEQ ID NO:3), LGALS3 (Galectin-3) (SEQ ID NO:8), CST2 (Cystatin SA) (SEQ ID NO:6), CAPC (Cytokeratin-Associated Protein In Cancer) (SEQ ID NO:9), GRP78 (78 kDa glucose-regulated protein) (SEQ ID NO:12) and GRN (Granulin) (SEQ ID NO:4), wherein at least one of the proteins is selected from the group consisting of A1AT, LGALS3, and CAPC.

2. The composition of claim 1, wherein the composition includes at least 3, 5, 6, 7, 8, or all 9 proteins or antigenic fragments thereof, selected from the group consisting of A1AT, ANGPTL4, LRP10, GFRA1, LGALS3, CST2, DKK1, CAPC, GRP78, and GRN.

3. The composition of any one of claims 1-2, wherein the composition consists of between 2 and 1000 proteins or antigenic fragments thereof.

4. The composition of any one of claims 1-3, wherein the composition includes at least 2, 3, 4, or all 5 of ANGPTL4, GFRA1, LGALS3, DKK1 and GRN proteins, or antigenic fragments thereof.

5. The composition of any one of claims 1-4, wherein the at least two proteins or antigenic fragments thereof comprise native extracellular domains and/or native secreted proteins or antigenic fragments thereof.

6. The composition of any one of claims 1-6, wherein the at least two proteins or antigenic fragments thereof are detectably labeled.

7. The composition of any one of claims 1-6, wherein the at least two proteins, or antigenic fragments thereof, are expressed as a fusion with a detectable domain, including but not limited to an Fc domain.

8. The composition of any one of claims 1-7, wherein the at least two proteins or antigenic fragments thereof are immobilized on a surface of a solid support.

9. A method for monitoring breast cancer therapy, comprising
(a) contacting a bodily fluid sample from a subject who is undergoing or has undergone breast cancer therapy with one or more proteins, or antigenic fragments thereof, selected from the group consisting of DKK1, A1AT, ANGPTL4, LRP10, GFRA1, LGALS3, CST2, CAPC, GRP78, and GRN; and
(b) determining an amount of autoantibodies against the one or more proteins in the bodily fluid sample;
wherein a decrease in the amount of autoantibodies relative to a control, such as a baseline level of autoantibodies in a similar bodily fluid sample from the subject indicates efficacy of the breast cancer therapy in the subject.

10. A method for prognosing breast cancer recurrence, comprising
(a) contacting a bodily fluid sample from a subject who has received breast cancer therapy with one or more proteins, or antigenic fragments thereof, selected from the group consisting of DKK1, A1AT, ANGPTL4, LRP10, GFRA1, LGALS3, CST2, CAPC, GRP78, and GRN; and
(b) determining an amount of autoantibodies against the one or more proteins in the bodily fluid sample;
wherein an increase in the amount of autoantibodies relative to a baseline level of autoantibodies in a control, such as a similar bodily fluid sample from the subject indicates a likelihood of breast cancer recurrence in the subject.

11. The method of any one of claims 9-10, wherein the one or more proteins, or antigenic fragments thereof, 3, 5, 6, 7, 8, or all 9 of the recited proteins, or antigenic fragments thereof.

12. The method of any one of claims 9-10, wherein the one or more proteins, or antigenic fragments thereof comprise at least 2, 3, 4, or all 5 proteins, or antigenic fragments thereof, selected from the group consisting of DKK1, ANGPTL4, GFRA1, LGALS3, and GRN.

13. The method of any one of claims 9-12, wherein the contacting comprises use of ELISA.

14. The method of any one of claims 9-13, wherein the bodily fluid sample comprises a blood sample, a plasma sample, or a serum sample from the subject.

15. The method of any one of claims 9-14, wherein the subject has had surgery to remove the primary tumor, and/or wherein the subject is receiving or has received radiation therapy and chemotherapy.

16. The method of any one of claims 9-15, wherein the contacting comprises use of Longitudinal Assay Screening, wherein all target biomarkers may be detected and quantitated within a single test and dilution.
